# FASCICULE DE BREVET EUROPEEN

(11) **EP 0 635 002 B1**
(45) Date de publication et mention de la délivrance du brevet: **22.07.1998**
(21) Numéro de dépôt: 93909004.9
(22) Date de dépôt: 08.04.1993
(51) Int. Cl.: C07D 209/44, A61K 31/40

(54) **NOUVEAUX DERIVES DE PERHYDROISOINDOLE, ET LEUR PREPARATION**
NEUE PERHYDROISOINDOLDERIVATE UND IHRE HERSTELLUNG
NOVEL PERHYDROISOINDOLE DERIVATIVES AND PREPARATION THEREOF

(30) Priorité: 10.04.1992 FR 9204391
(43) Date de publication de la demande: 25.01.1995
(73) Titulaire: RHONE-POULENC RORER S.A., 92160 Antony (FR)
(72) Inventeur: ACHARD, Daniel, F-94320 Thiais (FR); GRISONI, Serge, F-94600 Choisy-le-Roi (FR); MALLERON, Jean-Luc, F-91460 Marcoussis (FR); PEYRONEL, Jean-François, F-91120 Palaiseau (FR); TABART, Michel, F-75013 Paris (FR)
(74) Mandataire: Savina, Jacques
(86) Numéro de dépôt international: FR9300351
(87) Numéro de publication internationale: WO9321154

(56) Documents cités:
- EP-A- 0 429 366
- EP-A- 0 430 771
- EP-A- 0 514 274

## Description

La présente invention concerne de nouveaux dérivés du perhydroisoindole de formule générale : 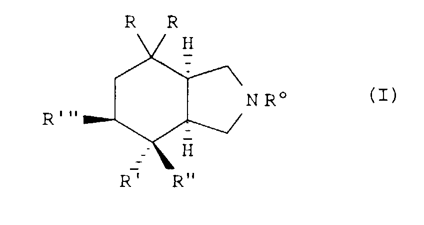 dans laquelle :
- les symboles R sont identiques et représentent des radicaux phényle éventuellement substitués par un atome d'halogène ou par un radical méthyle en position 2 ou 3,
- le symbole R' représente un radical phényle éventuellement substitué en position -2 par un radical alcoyle ou alcoyloxy contenant 1 ou 2 atomes de carbone,
- le symbole R" représente un atome de fluor ou un radical hydroxy, et le symbole R"' représente un atome d'hydrogène ou bien
- les symboles R" et R"' représentent des radicaux hydroxy, ou bien
- le symbole R" forme avec R"' une liaison, et
- le symbole R° représente un atome d'hydrogène ou représente un radical protecteur,
ainsi que leurs sels lorsqu'ils existent, qui sont des intermédiaires de synthèse pour la préparation de dérivés doués d'activité antagoniste de la substance P.

Dans la demande de brevet européen EP 430 771 ont été décrits des dérivés de perhydroisoindolone de structure : 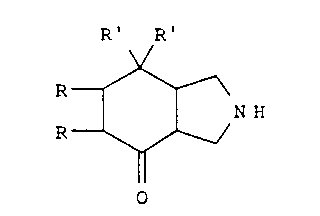 dans laquelle les symboles R sont hydrogène ou forment ensemble une liaison et les symboles R' sont des radicaux phényle éventuellement substitués qui sont des intermédiaires pour la préparation de perhydroisoindolones antagonistes de la substance P décrits dans la demande européenne EP 429 366. Cependant ces dérivés de perhydroisoindolones se sont montrés principalement actifs dans des tests de binding utilisant des homogénats de cerveau de rat et manifestent moins d'activité dans des tests de binding utilisant des cellules humaines lymphoblastiques en culture.

Dans le brevet américain 4 042 707 avaient été décrits des produits dérivés de l'isoindole de formule générale: 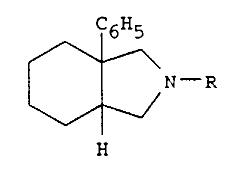 ayant une activité opiacée. Ces produits n'ont pas d'activité vis à vis de la substance P et ne sont pas non plus utiles comme intermédiaires.

Dans la formule générale (I), lorsque R porte un substituant halogène, ce dernier peut être choisi parmi le chlore ou le fluor.

Le radical protecteur R° est un groupement protecteur d'amino compatible et dont la mise en place et l'élimination n'altère pas le reste de la molécule choisi parmi les groupements alcoyloxycarbonyle, benzyloxycarbonyle, benzyle éventuellement substitués, formyle, chloracétyle, trichloracétyle, trifluoracétyle, vinyloxycarbonyle, phénoxycarbonyle, chloro-1 éthoxycarbonyle ou chlorocarbonyle.

Plus spécifiquement, les dérivés de perhydroisoindole pour lesquels R" est hydroxy ou fluor et R"' est hydrogène, de forme (3aS,4S,7aS) à l'état pur, ou sous forme de mélange racémique (3aRS,4RS,7aRS), les dérivés de perhydroisoindole pour lesquels R" et R"' sont hydroxy, de forme (3aS,4S,5S,7aS) à l'état pur, ou sous forme de mélange racémique (3aRS,4RS,5RS,7aRS), les dérivés de perhydroisoindole pour lesquels R" forme avec R"' une liaison, de forme (3aS,7aR) à l'état pur, ou sous forme de mélange racémique (3aRS,7aSR) font partie de la présente invention.

Selon l'invention, le dérivé de perhydroisoindole de formule générale (I) peut être obtenu par action d'un composé organométallique de formule générale :

R'-M (II)

dans laquelle R' est défini comme précédemment, et M représente le lithium, un radical MgX ou CeX₂ pour lequel X est un atome d'halogène, sur le dérivé correspondant de perhydroisoindolone de formule générale : 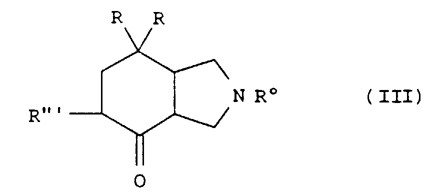 dans laquelle R est défini comme précédemment, R"' est un atome d'hydrogène ou un radical hydroxy éventuellement protégé, et R° est un radical protecteur tel que défini précédemment, puis le cas échéant libération du radical protecteur de R"', puis éventuellement transformation du produit obtenu pour lequel R" est un radical hydroxy et R"' est un atome d'hydrogène, en un produit pour lequel R" est un atome de fluor et R"' est un atome d'hydrogène ou en un produit pour lequel R" et R"' forment ensemble une liaison et éventuellement élimination du radical protecteur R°.

La protection du radical R"' et l'élimination du radical protecteur s'effectuent selon les méthodes habituelles de protection et/ou d'élimination des radicaux hydroxy, qui n'altèrent pas le reste de la molécule.

La réaction s'effectue en milieu anhydre, dans les conditions habituelles de réaction des composés organométalliques sur une cétone, qui n'affectent pas le reste de la molécule. Notamment on opère dans un éther (par exemple le tétrahydrofuranne ou l'éther éthylique) éventuellement en présence de chlorure de cérium anhydre à une température comprise entre -78 et 30°C. Il est entendu que selon la nature du radical protecteur du radical R"', ce dernier peut être éliminé simultanément à la réaction.

Le dérivé de l'isoindole de formule générale (I) pour lequel R" est un atome de fluor et R"' est un atome d'hydrogène peut être préparé par fluoration d'un dérivé de l'isoindole de formule générale (I) pour lequel R et R' sont définis comme précédemment, R° est un radical protecteur, R" est un radical hydroxy et R"' est un atome d'hydrogène, puis éventuellement élimination du radical protecteur R°.

La réaction s'effectue avantageusement au moyen d'un agent de fluoration comme un fluorure de soufre [trifluorure de morpholino soufre, tétrafluorure de soufre (J. Org. Chem., 40, 3808 (1975)), trifluorure de diéthylamino soufre (Tetrahedron, 44, 2875 (1988)), trifluorure de phényl soufre (J. Am. Chem. Soc., 84, 3058 (1962)], comme le tétrafluorure de sélénium (J. Am. Chem. Soc., 96,925 (1974) ou comme le tétrafluorophénylphosphorane (Tet. Let., 907 (1973), en opérant dans un solvant organique tel qu'un solvant chloré (dichlorométhane, dichloréthane par exemple) à une température comprise entre -30 et 30°C.

Le dérivé de perhydroisoindole de formule générale (I) pour lequel R" et R"' forment ensemble une liaison peut être obtenu par deshydratation du dérivé correspondant de perhydroisoindole de formule générale (I) pour lequel R" est un radical hydroxy et R"' est un atome d'hydrogène et R et R° sont définis comme précédemment, puis éventuellement élimination du radical protecteur R°.

On opère par toute méthode connue de deshydratation des alcools qui n'altère pas le reste de la molécule. Notamment on effectue la deshydratation en milieu acide par exemple par action d'un acide sulfonique (acide p.toluènesulfonique...), de l'acide sulfurique, de l'acide phosphorique, du pentaoxyde de phosphore ou de l'oxyde d'aluminium ou par action d'un mélange acide chlorhydrique-acide acétique ou acide bromhydrique-acide acétique, à une température comprise entre 25°C et la température de reflux du mélange réactionnel.

Lorsque l'on veut obtenir un produit de formule générale (I) pour lequel R° est un atome d'hydrogène, l'élimination subséquente du radical protecteur R° s'effectue selon les méthodes habituelles. Notamment selon les méthodes décrites par T.W. Greene, par A. Wiley ou par Mc Omie dans les références citées précédemment.

Le dérivé de la perhydroisoindolone de formule générale (III) pour laquelle R"' est un atome d'hydrogène de forme (3aRS,7aRS) peut être préparé selon la méthode décrite dans la demande de brevet européen EP 430 771. La séparation du stéréoisomère (3aS,7aS) s'effectue par analogie avec les méthodes décrites dans cette demande et selon la méthode décrite ci-après dans les exemples. Le dérivé de la perhydroisoindolone de formule générale (III) pour lequel R"' est un radical hydroxy préalablement protégé peut être aussi préparé par analogie avec cette méthode, ou comme décrit ci-après dans les exemples.

D'une manière générale, lorsque l'on veut obtenir un produit de formule générale (I) de forme (3aS,7aS), la séparation des formes isomères est mise en oeuvre de préférence au niveau du dérivé de formule générale (III) ou au niveau d'un autre intermédiaire portant un radical oxo en position -4. Néanmoins elle peut aussi être mise en oeuvre au niveau du dérivé de formule générale (I). La séparation s'effectue selon toute méthode connue et compatible avec la molécule.

A titre d'exemple, la séparation peut être effectuée par préparation d'un sel optiquement actif, par action de l'acide L(+) ou D(-) mandélique, ou de l'acide dibenzoyltartrique ou ditoluoyltartrique, puis séparation des isomères par cristallisation. L'isomère recherché est libéré de son sel en milieu basique.

Les nouveaux dérivés de l'isoindole de formule générale (I) sont particulièrement intéressants comme intermédiaires pour la préparation de produits de formule générale : 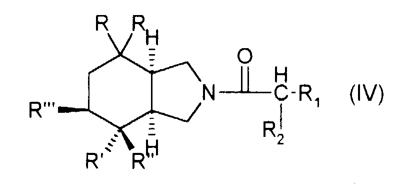 dans laquelle R, R', R" et R"' sont définis comme précédemment, et le symbole R₁ représente un radical phényle éventuellement substitué par un ou plusieurs atomes d'halogène ou radicaux hydroxy, alcoyle pouvant être éventuellement substitués (par des atomes d'halogène ou des radicaux amino, alcoylamino ou dialcoylamino) alcoyloxy ou alcoylthio pouvant être éventuellement substitues [par des radicaux hydroxy, amino, alcoylamino ou dialcoylamino éventuellement substitués (par des radicaux phényle, hydroxy ou amino), ou dialcoylamino dont les parties alcoyle forment avec l'atome d'azote auquel elles sont rattachées, un hétérocycle à 5 à 6 chaînons pouvant contenir un autre hétéroatome choisi parmi l'oxygène, le soufre ou l'azote, éventuellement substitué par un radical alcoyle, hydroxy, hydroxyalcoyle)], ou substitué par des radicaux amino, alcoylamino, dialcoylamino dont les parties alcoyle peuvent former avec l'atome d'azote auquel elles sont rattachées, un hétérocycle tel que défini ci-dessus, ou représente un radical cyclohexadiènyle, naphtyle ou hétérocyclyle mono ou polycyclique, saturé ou insaturé contenant 5 à 9 atomes de carbone et un ou plusieurs hétéroatomes choisis parmi l'oxygène, l'azote ou le soufre, et éventuellement substitué par un atome d'halogène ou par un radical alcoyle ou alcoyloxy, et le symbole R₂ représente un atome d'hydrogène ou d'halogéné ou un radical hydroxy, alcoyle, aminoalcoyle, alcoylaminoalcoyle, dialcoylaminoalcoyle, alcoyloxy, alcoylthio, acyloxy, carboxy, alcoyloxycarbonyle, dialcoylaminoalcoyloxycarbonyle, benzyloxycarbonyle, amino ou acylamino, sous forme racémique,
sous ses formes (R) ou (S) sur la chaîne -CHR₁R₂, ou sous forme du mélange de plusieurs de ces formes, ainsi que leurs sels, étant entendu que les radicaux alcoyle ou acyle cités ci-dessus contiennent (sauf mention spéciale) 1 à 4 atomes de carbone en chaîne droite ou ramifiée.

Ces dérives de perhydroisoindole sont particulièrement intéressants comme antagonistes de la substance P.

Dans la formule générale (IV), lorsque R₁ contient un atome d'halogène, ce dernier peut être choisi parmi le chlore, le brome, le fluor ou l'iode.

Dans la formule générale (IV), lorsque R₁ représente un radical hétérocyclyle mono ou polycyclique, saturé ou insaturé, à titre d'exemple il peut être choisi parmi thiényle, furyle, pyridyle, dithiinyle, indolyle, isoindolyle, benzothiényle, thiazolyle, isothiazolyle, oxazolyle, imidazolyle, pyrrolyle, triazolyle, thiadiazolyle, quinolyle, isoquinolyle, ou naphtyridinyle.

Dans la formule générale (IV), lorsque R₁ représente phényle substitué par une chaîne portant un hétérocycle, ce dernier peut être choisi parmi pyrrolidinyle, morpholino, pipéridinyle, tétrahydropyridinyle, pipérazinyle, thiomorpholino.

De plus, lorsque le symbole R₂ est autre que l'atome d'hydrogène, la chaîne substituée sur l'isoindole présente un centre chiral, il est entendu que les formes stéréoisomères et leurs mélanges entrent aussi dans la formule générale (IV).

Les dérivés de perhydroisoindole de formule générale (IV) peuvent être préparés à partir des produits selon l'invention en opérant de la manière suivante :
on fait agir l'acide de formule générale : 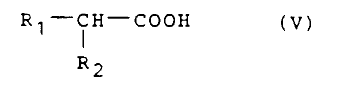 ou un dérivé réactif de cet acide, dans lequel R₁ et R₂ sont définis comme précédemment, sur un dérivé de l'isoindole de formule générale (I) pour lequel R° est un atome d'hydrogène, puis éventuellement transforme le produit obtenu pour lequel R" est un radical hydroxy et R"' est un atome d'hydrogène en un produit pour lequel R" est un atome de fluor et R"' est un atome d'hydrogène ou en un produit pour lequel R" et R"' forment ensemble une liaison.

Il est entendu que, les radicaux amino, alcoylamino ou carboxy contenus dans R₁ et/ou R₂ sont de préférence préalablement protégés. La protection s'effectue par tout groupement compatible, dont la mise en place et l'élimination n'affectent pas le reste de la molécule. Notamment, on opère selon les méthodes décrites par T.W. Greene, Protective Groups in Organic Synthesis, A. Wiley - Interscience Publication (1981), ou par Mc Omie, Protective Groups in Organic Chemistry, Plenum Press (1973).

A titre d'exemple,
- les groupements amino ou alcoylamino peuvent être protégés par des radicaux méthoxycarbonyle, éthoxycarbonyle, t.butoxycarbonyle, allyloxycarbonyle, vinyloxycarbonyle, trichloréthoxycarbonyle, trichloracétyle, trifluoracétyle, chloracétyle, trityle, benzhydryle, benzyle, allyle, formyle, acétyle, benzyloxycarbonyle ou ses dérivés substitués;
- les groupements acides peuvent être protégés par des radicaux méthyle, éthyle, t.butyle, benzyle, benzyle substitué ou benzhydryle.

De plus, lorsque R₂ représente un radical hydroxy, il est préférable de protéger préalablement ce radical. La protection s'effectue par exemple par un radical acétyle, trialcoylsilyle, benzyle, sous forme d'un carbonate par un radical -COORa dans lequel Ra est un radical alcoyle ou benzyle ou sous forme de cétone.

Lorsque l'on effectue la condensation d'un dérivé réactif de l'acide de formule générale (V), on opère avantageusement au moyen du chlorure d'acide, de l'anhydride, d'un anhydride mixte ou d'un ester réactif dans lequel le reste de l'ester est un radical succinimido, benzotriazolyl-1 éventuellement substitué, nitro-4 phényle, dinitro-2,4 phényle, pentachlorophényle ou phtalimido.

La réaction s'effectue généralement à une température comprise entre -40 et +40°C, dans un solvant organique tel qu'un solvant chloré (dichlorométhane, dichloréthane, chloroforme par exemple), un hydrocarbure (toluène par exemple), un éther (tétrahydrofuranne, dioxanne par exemple), un ester (acétate d'éthyle par exemple), un amide (diméthylacétamide, diméthylformamide par exemple), ou une cétone (acétone par exemple) ou dans un mélange de ces solvants, en présence d'un accepteur d'acide tel qu'une base organique azotée comme par exemple la pyridine, la diméthylaminopyridine, la N-méthylmorpholine ou une trialcoylamine (notamment triéthylamine) ou tel qu'un époxyde (oxyde de propylène par exemple). Il est également possible d'opérer en présence d'un agent de condensation tel qu'un carbodiimide, [par exemple dicyclohexylcarbodiimide ou (diméthylamino-3 propyl)-1 éthyl-3 carbodiimide], le NN'-carbonyldiimidazole ou l'éthoxy-2 éthoxycarbonyl-1 dihydro-1,2 quinoléine ou bien en milieu hydroorganique, en présence d'un agent alcalin de condensation comme le bicarbonate de sodium.

Dans l'alternative où l'on a obtenu un dérivé de perhydroisoindole de formule générale (IV) pour lequel R" est un radical hydroxy et R"' est un atome d'hydrogène et où l'on veut obtenir un dérivé de perhydroisoindole pour lequel R" est un atome de fluor et R"' est un atome d'hydrogène, on opère par fluoration du dérivé précédemment obtenu.

La fluoration s'effectue dans les conditions décrites précédemment pour la fluoration d'un dérivé de formule générale (I) dans laquelle R" est hydroxy, à une température comprise entre -30 et +30°C.

Dans l'alternative où l'on a obtenu un dérivé de perhydroisoindole de formule générale (IV) pour lequel R" est un radical hydroxy et R"' est un atome d'hydrogène et où l'on veut obtenir un dérivé de perhydroisoindole pour lequel R" et R"' forment ensemble une liaison, on opère par deshydratation du dérivé précédemment obtenu.

La réaction s'effectue dans les conditions décrites précédemment pour la préparation des dérivés de formule générale (I) dans laquelle R" et R"' forment ensemble une liaison à partir du dérivé correspondant de perhydroisoindole pour lequel R" est un radical hydroxy et R"' est un atome d'hydrogène.

Les acides de formule générale (V) peuvent être préparés selon les méthodes décrites ci-après dans les exemples, selon les méthodes décrites dans la demande de brevet EP 429 366 ou par analogie avec ces méthodes.

Les nouveaux dérivés de l'isoindole de formule générale (I) ou les dérivés de formule générale (IV) peuvent être purifiés le cas échéant par des méthodes physiques telles que la cristallisation ou la chromatographie.

Le cas échéant, les nouveaux dérivés de formule générale (I) pour lesquels R° est un atome d'hydrogène ou les produits de formule générale (IV) pour lesquels les symboles R₁ et/ou R₂ contiennent des substituants amino ou alcoylamino, peuvent être transformés en sels d'addition avec les acides. Comme exemples de sels d'addition avec des acides peuvent être cités les sels formés avec les acides minéraux (chlorhydrates, bromhydrates, sulfates, nitrates, phosphates) ou avec les acides organiques (succinates, fumarates, tartrates, acétates, propionates, maléates, citrates, méthanesulfonates, p.toluènesulfonates, iséthionates, ou avec des dérivés de substitution de ces composés).

D'un intérêt particulier sont les produits de formule générale (I) pour lesquels :
les symboles R sont identiques et représentent des radicaux phényle,
le symbole R' représente un radical phényle éventuellement substitué en position -2 par un radical alcoyle ou alcoyloxy contenant 1 ou 2 atomes de carbone,
le symbole R" représente un atome de fluor ou un radical hydroxy, et le symbole R"' représente un atome d'hydrogène ou bien les symboles R" et R"' représentent des radicaux hydroxy, ou bien le symbole R" forme avec R"' une liaison, et
le symbole R° représente un atome d'hydrogène ou représente un radical benzyle ou t.butoxycarbonyle.

Et parmi ces produits plus particulièrement les :
- diphényl-7,7 (méthoxy-2 phényl)-4 tert-butoxycarbonyl-2 perhydroisoindolol-4;
- diphényl-7,7 (méthoxy-2 phényl)-4 perhydroisoindolol-4;
- diphényl-7,7 (méthoxy-2 phényl)-4 tert-butoxycarbonyl-2 perhydroisoindolediol-4,5;
- diphényl-7,7 (méthoxy-2 phényl)-4 perhydroisoindolediol-4,5;
- benzyl-2 diphényl-7,7 (méthoxy-2 phényl)-4 perhydroisoindolediol-4,5.

Les dérivés de l'isoindole de formule générale (IV) qui antagonisent les effets de la substance P peuvent trouver une application dans les domaines de l'analgésie, de l'inflammation de l'asthme, des allergies, sur le système nerveux central, sur le système cardiovasculaire, comme antispasmodique, ou sur le système immunitaire, ainsi que dans le domaine de la stimulation des sécrétions lachrymales.

En effet, les produits de formule générale (IV) manifestent une affinité pour les récepteurs à substance P à des doses comprises entre 10 et 1000 nM selon les techniques adaptées de D.G. Payan et coll., J. of immunology, 133(6), 3260-5 (1984) : Stereospecific receptors for substance P on cultured human IM-9 lymphoblasts et de Mc Pherson et coll., J. Pharmacol. Meth., 14, 213 (1985) : Analysis of radioligand binding experiments.

Il a de plus été démontré qu'il s'agit d'un effet antagoniste de la substance P, au moyen de différents produits. Dans la technique décrite par S. Rosell et coll., Substance P, Ed. by US Von Euler and B. Pernow, Raven Press, New-York (1977), pages 83 à 88, les produits étudiés manifestent un antagonisme des contractions de l'iléon de cobaye induites par la substance P ou des contractions de l'iléon de cobaye induites par le septide, à des concentrations de 6 à 1000 nM.

La substance P est connue pour être impliquée dans un certain nombre de domaines pathologiques :
- Agonists and antagonists of substance P, A.S. Dutta Drugs of the futur, 12 (8), 782 (1987);
- Substance P and pain : an updating, J.L. Henry, TINS, 3(4), 97 (1980);
- Substance P in inflammatory reactions and pain, S. Rosell, Actual. Chim. Ther., 12ème série, 249 (1985)
- Effects of Neuropeptides on Production of Inflammatory Cytokines by Human Monocytes, M. Lotz et coll., Science, 241, 1218 (1988).
- Neuropeptides and the pathogenesis of allergy, Allergy, 42, 1 à 11 (1987);
- Substance P in Human Essential Hypertension, J. Cardiocascular Pharmacology, 10 (suppl. 12), 5172 (1987).

L'étude de certains dérivés de l'isoindole de formule générale (IV) dans la technique de A. Saria et coll., Arch. Pharmacol.,324, 212-218 (1983) adaptée au cobaye a permis de mettre en évidence un effet inhibiteur de l'augmentation de la perméabilité capillaire entraîné par le septide (agoniste de la substance P), ce qui témoigne d'une activité anti-inflammatoire :

| Produit étudié | DE₅₀ |
|---|---|
| Exemple d'utilisation 1 | 0,04 mg/kg i.v. |
| | 3,5 mg/kg p.o. |

L'injection de substance P chez l'animal provoque une hypotension. Les produits étudiés dans la technique de C.A. Maggi et coll., J. Auton. Pharmac., 7, 11-32 (1987) manifestent un effet antagoniste vis-à-vis de cette hypotension, chez le cobaye. On détermine la DE₅₀ dose qui diminue de 50% l'hypotension induite par une injection i.v. de 250 ng/kg de substance P.

| Produit de formule générale (I) | DE₅₀ mg/kg i.v. |
|---|---|
| Exemple d'utilisation 1 | 0,15 |

L'injection de substance P provoque chez l'animal un bronchospasme. La bronchoconstriction induite in vivo chez le cobaye par l'injection de substance P ou d'un agoniste sélectif de la substance P : [Pro⁹] substance P, est étudiée selon la technique de H. Konzett et R. Rosseler, Archiv. Exp. Path. Pharmak., 195, 71-74 (1940). Cette bronchoconstriction est inhibée par l'injection d'un produit de formule générale (IV), ce qui témoigne d'une activité anti-asthmatique. On détermine la DE₅₀, dose qui diminue de 50% le bronchospasme induit par 3 µg/kg i.v. de [Pro⁹] substance P. Dans cette technique la DE₅₀ du produit de l'exemple d'utilisation 1 est de 0,7 mg/kg i.v..

Par ailleurs, les dérivés de l'isoindole de formule générale (IV) ne présentent pas de toxicité, ils se sont montrés atoxiques chez la souris par voie intra-veineuse à la dose de de 10 mg/kg ou par voie sous cutanée à la dose de 40 mg/kg par voie sous-cutanée.

Les exemples suivants donnés à titre non limitatif illustrent la présente invention.

Dans les exemples qui suivent, il est entendu que, sauf mention spéciale, les spectres de RMN du proton ont été faits à 250 MHz dans le diméthylsulfoxyde; les déplacements chimiques sont exprimés en ppm.

### Exemple 1

A une suspension de 20 g de diphényl-7,7 tert-butoxycarbonyl-2 perhydroisoindolone-4-(3aS,7aS) et de 31,6 g de chlorure de cérium anhydre dans 250 cm³ de tétrahydrofuranne sec, on ajoute goutte à goutte à température ambiante sous agitation une suspension de bromure de méthoxy-2 phénylmagnésium (préparée à partir de 75,3 g de bromo-2 anisole et de 9,8 g de magnésium) dans 100 cm³ de tétrahydrofuranne sec. Le mélange réactionnel est agité à température ambiante pendant 24 heures, traité par 400 cm³ d'une solution aqueuse saturée en chlorure d'ammonium, dilué par 200 cm³ d'acétate d'éthyle, lavé par 300 cm³ d'eau (deux fois), puis par 300 cm³ d' une solution aqueuse saturée en chlorure de sodium, séché sur sulfate de magnésium, filtré et concentré à sec sous pression réduite (2,7 kPa). Le résidu est chromatographié sur colonne de gel de silice Merck (granulométrie 0,04-0,06 mm, diamètre 5,8 cm, hauteur 26,5 cm), en éluant sous une pression de 0,5 bar d'azote par un mélange de cyclohexane et d'acétate d'éthyle (80/20 en volumes) et en recueillant des fractions de 100 cm³. Les fractions 9 à 29 sont réunies et concentrées à sec sous pression réduite (2,7 kPa). On obtient 17,82 g de diphényl-7,7 (méthoxy-2 phényl)-4 tert-butoxycarbonyl-2 perhydroisoindolol-4-(3aS,4S,7aS) sous forme d'une meringue blanche.
Spectre RMN du proton (DMSO d₆): 1,36 (s, 9H, C(CH₃)₃); 1,54 (dmt, J=14, 1H, H équatorial du -CH₂- en 5); 2,3 (dmt, J=14, 1H, H équatorial du -CH₂- en 6); 2,34 (td, J=14 et 2,5, 1H, H axial du -CH₂- en 5); 3,07(td, J=14 et 2,5, H axial du -CH₂-en 6); 3,49(s, 3H, -OCH₃); 2,6 à 3,6(mt, autre -CH₂- et -CH); 6,85 à 7,7 (mt, 14H, aromatiques).

A une solution de 7,63 g de diphényl-7,7 (méthoxy-2 phényl)-4 tert-butoxycarbonyl-2 perhydroisoindolol-4-(3aS,4S,7aS) dans 66 cm³ de dioxanne on ajoute à température ambiante une solution de 100 cm³ de dioxanne chlorhydrique 5,2 N. Le mélange réactionnel est agité 1 heure à cette température, puis concentré à sec sous pression réduite (2,7 kPa). Le résidu est lavé par de l'acétonitrile, essoré puis séché. On obtient 4,88 g de chlorhydrate de diphényl-7,7 (méthoxy-2 phényl)-4 perhydroisoindolol-4-(3aS,4S,7aS) sous la forme de cristaux blancs fondant à 271°C (bloc Maquenne).

La diphényl-7,7 tert-butoxycarbonyl-2 perhydroisoindolone-4-(3aS,7aS) peut être obtenue de la manière suivante :

A une suspension de 80 g de chlorhydrate de diphényl-7,7 perhydroisoindolone-4-(3aS,7aS) dans 400 cm³ de dichlorométhane sec on ajoute à température ambiante sous agitation 34,3 cm³ de triéthylamine, 58,6 g de di-tert-butyl dicarbonate, puis 2,98 g de diméthylamino-4 pyridine. Le mélange réactionnel est agité à température ambiante pendant 24 heures, lavé par 100 cm³ d'une solution aqueuse d'acide citrique, puis par 100 cm³ d'une solution aqueuse d'hydrogénocarbonate de sodium, puis par 100 cm³ d'une solution aqueuse saturée en chlorure de sodium, séché sur sulfate de magnésium, et concentré à sec sous pression réduite (2,7 kPa). On obtient 106,5 g de diphényl-7,7 tert-butoxycarbonyl-2 perhydroisoindolone-4-(3aS,7aS) sous la forme d'une meringue orange.

Spectre RMN du proton (DMSO d₆): 1,4(s, 9H, -C(CH₃)₃); 2,11(td, J=15 et 7,5, 1H, H axial du -CH₂-en 5); 2,3 (dt, J-15 et 3,5, 1H, H équatorial du -CH₂- en 5); 2,75 à 2,9 (mt, 4H, -CH₂- en 6 et -CH₂- en 1); 3,26 (dd, J=7,5 et 7, 1H, -CH en 3a); 3,35 (dd, J=11 et 7, 1H, 1H du -CH₂- en 3); 3,97 (mt, 1H, -CH en 7a); 4,1(d, J=11, 1H, l'autre H du -CH₂- en 3); 7,1 à 7,7 (mt, 10H, aromatiques).

Le chlorhyrate de diphényl-7,7 perhydroisoindolone-4-(3aS,7aS) peut être obtenu de la manière suivante :

A une suspension de 20 g de chlorhydrate de diphényl-7,7 perhydroisoindolone-4-(3aRS,7aRS) dans 250 cm³ d'acétate d'éthyle, on ajoute lentement, sous agitation, 50 cm³ de soude aqueuse 4N; l'agitation est poursuivie jusqu'à disparition du produit de départ. La solution organique est lavée par 100 cm³ d'eau distillée, par 100 cm³ d'une solution saturée en chlorure de sodium, séchée sur sulfate de magnésium et filtrée. A la solution ainsi obtenue, on ajoute sous agitation une solution de 9,3 g d'acide D-(-) mandélique dans 50 cm³ d'acétate d'éthyle. Les cristaux formés sont filtrés essorés, lavés par 50 cm³ d'acétate d'éthyle (deux fois) et séchés. Les cristaux sont repris par une solution de 220 cm³ d'acétonitrile et de 60 cm³ d'eau distillée, le mélange est porté au reflux sous agitation pendant 15 minutes; les cristaux formés sont filtrés, et à nouveau cristallisés dans un mélange de 100 cm³ d'acétonitrile et de 35 cm³ d'eau distillée. On obtient 6,4 g de D-mandélate de diphényl-7,7 perhydroisoindolone-4-(3aS,7aS).

A 6,4 g de D-mandélate de diphényl-7,7 perhydroisoindolone-4-(3aS,7aS) en solution dans 100 cm³ d'acétate d'éthyle on ajoute 50 cm³ de soude aqueuse 1N; le mélange réactionnel est agité à température ambiante jusqu'à disparition du produit de départ; la solution organique est lavée par 50 cm³ d'eau distillée, par 50 cm³ d'une solution aqueuse saturée en chlorure de sodium, séchée sur sulfate de magnésium et filtrée; elle est acidifiée sous agitation par addition de 2 cm³ d'une solution 9N d'acide chlorhydrique dans l'éthanol; les cristaux obtenus sont essorés, lavés par de l'acétate d'éthyle, puis par de l'oxyde d'isopropyle et séchés. On obtient 4,24 g de chlorhydrate de diphényl-7,7 perhydroisoindolone-4-(3aS,7aS) sous la forme de cristaux blancs fondant à 270°C avec décomposition.

L'acide (méthoxy-2 phényl)-2 propionique-(S) peut être préparé, par analogie avec les méthodes décrites par D.A Evans et coll., Tetrahedron, 44, 5525, (1988), selon le mode opératoire suivant:

A une solution refroidie à +5°C de 4,1 g de méthyl-4 phényl-5 [(méthoxy-2 phényl)-2-(S)-propionyl]-3 oxazolidinone-2-(4S,5S) dans 60 cm³ de tétrahydrofuranne et 30 cm³ d'eau, on ajoute 1,52 g d'hydroxyde de lithium. Le mélange réactionnel est agité 3 heures à cette tempétature, puis, après retour à température ambiante, on rajoute de l'acétate d'éthyle, on décante, la phase aqueuse est acidifiée par une solution aqueuse d'acide chlorhydrique 1N, extraite par de l'acétate d'éthyle, la phase organique est séchée sur sulfate de magnésium, et concentrée à sec sous pression réduite (2,7 kPa). Le solide obtenu est recristallisé dans l'hexane, essoré et séché. On obtient 0,4 g d'acide (méthoxy-2 phényl)-2 propionique-(S) sous la forme de cristaux blancs fondant à 102°C. [α]_{D}²⁰ = +84,6° (c=1; CHCl₃).

La méthyl-4 phényl-5 [(méthoxy-2 phényl)-2-(S)-propionyl]-3 oxazolidinone-2-(4S,5S)peut être obtenue de la manière suivante :

A une solution refroidie à -50°C de 10 g de méthyl-4 phényl-5 [(méthoxy-2 phényl)-acétyl]-3 oxazolidinone-2-(4S,5S) dans 150 cm³ de tétrahydrofuranne on ajoute 19,1 g d'hexaméthyl-1,1,1,3,3,3 disilazanate de sodium, on agite 45 minutes à cette température, puis on ajoute 7,72 cm³ d'iodure de méthyle. Le mélange réactionnel est ensuite agité 15 heures à température ambiante, puis dilué par de l'acétate d'éthyle, lavé par 50 cm³ d'eau, puis par 50 cm³ d'une solution aqueuse saturée de chlorure de sodium, séché sur sulfate de magnésium et concentré à sec sous pression réduite (2,7 kPa). Le résidu obtenu est cristallisé dans l'oxyde d'isopropyle, essoré et séché. On obtient 4,2 g de méthyl-4 phényl-5 [(méthoxy-2 phényl)-2-(S)-propionyl]-3 oxazolidinone-2- (4S,5S) sous la forme d'un solide blanc.

La méthyl-4 phényl-5 (méthoxy-2 phénylacétyl)-3 oxazolidinone-2-(4S,5S) peut être obtenue de la manière suivante :

A une suspension de 1,89 d'hydrure de sodium (dispersion à 80% dans la vaseline) dans 200 cm³ de tétrahydrofuranne sec on ajoute à température ambiante 9,38 g d'acide méthoxy-2 phénylacétique. On refroidit cette suspension à -30°C, on ajoute 7,77 cm³ de chlorure de pivaloyle, puis on ajoute enfin une solution refroidie à -78°C obtenue en additionnant une solution de 35,27 cm³ de butyllithium 1,6 M dans l'hexane à une solution refroidie à -78°C de 10 g de méthyl-4 phényl-5 oxazolidinone-2-(4S,5S) dans 200 cm³ de tétrahydrofuranne sec. Le mélange réactionnel est agité 45 minutes à -30°C, puis après retour à température ambiante, on ajoute 200 cm³ d'une solution aqueuse saturée de chlorure d'ammonium, puis 500 cm³ d'acétate d'éthyle; aprés décantation la phase organique est lavée deux fois par 100 cm³ d'eau, puis deux fois par 100 cm³ d'une solution aqueuse saturée de chlorure de sodium; séchée sur sulfate de magnésium et concentrée à sec sous pression réduite (2,7 kPa). Le résidu est chromatographié sur colonne de gel de silice (granulométrie 0,04-0,06 mm, diamètre 4,8 cm, hauteur 36 cm), en éluant sous une pression de 0,6 bar d'azote par un mélange de cyclohexane et d'acétate d'éthyle (85/15 puis 80/20 en volumes) et en recueillant des fractions de 50 cm³. Les fractions 14 à 31 sont réunies et concentrées à sec sous pression réduite (2,7 kPa). On obtient 13,6 g de méthyl-4 phényl-5 (méthoxy-2 phénylacétyl)-3 oxazolidinone-2-(4S,5S) sous la forme d'une huile jaune.

### Exemple 2

En opérant selon le mode opératoire de l'exemple 1 à partir de 3 g de diphényl-7,7 tert-butoxycarbonyl-2 perhydroisoindolone-4-(3aS,7aS) et d'une suspension de bromure de méthyl-2 phénylmagnésium (préparée à partir de 4,6 cm³ de bromo-2 toluène et de 0,93 g de magnésium dans 15 cm³ de tétrahydrofuranne anhydre) on obtient 1,5 g de diphényl-7,7 (méthyl-2 phényl)-4 tert-butoxycarbonyl-2 perhydroisoindolol-4-(3aS,4S,7aS) sous forme d'une huile utilisée telle quelle dans l'essai suivant.

Le chlorhydrate de diphényl-7,7 (méthyl-2 phényl)-4 perhydroisoindolol-4-(3aS,4S,7aS) peut être préparé de la manière suivante:

En opérant selon l'exemple 1 à partir de 1,2 g de diphényl-7,7 (méthyl-2 phényl)-4 tert-butoxycarbonyl-2 perhydroisoindolol-4-(3aS,4S,7aS) on obtient 0,68 g de chlorhydrate de diphényl-7,7 (méthyl-2 phényl)-4 perhydroisoindolol-4-(3aS,4S,7aS).

Spectre infra-rouge (KBr), bandes caractéristiques (cm⁻¹): 3325, 3100-3000, 3000-2850, 3000-2300, 1600, 1585, 1560, 1495, 1445, 750, 700.

### Exemple 3

En opérant selon le mode opératoire de l'exemple 1 à partir de 2,75 g de diphényl-7,7 tert-butoxycarbonyl-2 perhydroisoindolone-4-(3aRS,7aRS), de 1,73 g de chlorure de cérium anhydre et d'une suspension de bromure de méthoxy-2 phénylmagnésium (obtenue à partir de 6,57 g de bromo-2 anisole et de 0,84 g de magnésium) on obtient 2,72 g de diphényl-7,7 (méthoxy-2 phényl)-4 tert-butoxycarbonyl-2 perhydroisoindolol-4-(3aRS,4RS,7aRS) sous forme d'une meringue blanche.

Spectre RMN du proton (DMSO d₆): A température ambiante, on observe le mélange des rotamères: 1,3 et 1,35 (mt, 1H, 1H du -CH₂- en 5); 2,15 à 2,4 (mt, 2H, l'autre H du -CH₂- en 5 et 1H du -CH₂- en 6); 2,5 à 3,6 (mt, -CH₂- et -CH<); 3,35 et 3,39 (2s, 3H, -OCH₃); 4,68 et 4,72 (2s, 1H, -OH); 6,8 à 7,7 (mt, 14H aromatiques).

En opérant selon le mode opératoire de l'exemple 1 à partir de 2,7 g de diphényl-7,7 (méthoxy-2 phényl)-4 tert-butoxycarbonyl-2 perhydro isoindolol-4-(3aRS,4RS,7aRS) on obtient 1,77 g de chlorhydrate de diphényl-7,7 (méthoxy-2 phényl)-4 perhydroisoindolol-4-(3aRS,4RS,7aRS) sous la forme d'un solide blanc.

Spectre RMN du proton (DMSO d₆): 1,55 (d large, J=14, 1H, H équatorial du -CH₂- en 5); 2,34 (td, J=14 et 2,5, 1H, H axial du -CH₂- en 5); 2,37 (d large, J=14, 1H, H équatorial du -CH₂- en 6); 2,52 (mt, 1H du -CH₂- en 1); 2,93 (td, J=14 et 2,5; 1H, H axial du -CH₂- en 6); 3 à 3,3 (mt, 3H, -CH₂-en 3 et l'autre H du -CH₂- en 1); 3,42 (s, 3H,-OCH₃-); 3,4 à 3,7 (mt, 2H, -CH< en 3a et 7a); 5,3 (mf étalé, 1H, - OH); 6,8 à 7,7 (mt, 14H, aromatiques).

La diphényl-7,7 tert-butoxycarbonyl-2 perhydroisoindolone-4-(3aRS,7aRS) peut être préparée de la manière suivante :

A une suspension de 10 g de chlorhydrate de diphényl-7,7 perhydroisoindolone-4-(3aRS,7aRS) dans 50 cm³ de dichlorométhane sec on ajoute à température ambiante sous agitation 4,3 cm³ de triéthylamine, 7,4 g de di-tert-butyl dicarbonate, puis 0,37 g de diméthylamino-4 pyridine. Le mélange réactionnel est agité à température ambiante pendant 24 heures, lavé par 150 cm³ d'une solution aqueuse d'acide citrique, puis par 100 cm³ d'une solution aqueuse d'hydrogénocarbonate de sodium, puis par 100 cm³ d'une solution aqueuse saturée en chlorure de sodium, séché sur sulfate de magnésium, et concentré à sec sous pression réduite (2,7 kPa). On obtient 11 g de diphényl-7,7 tert-butoxycarbonyl-2 perhydroisoindolone-4-(3aRS,7aRS) sous la forme d'une meringue crème.

Spectre RMN du proton (DMSO d₆): 1,38 (s, 9H, -C(CH₃)₃); 2,08 (td, J=14 et 6, 1H, H axial du -CH₂- en 5); 2,28 (dmt, 1H, H équatorial du -CH₂- en 5); 2,7 à 2,85 (mt, 4H, -CH₂- 1 et -CH₂- en 6); 3,27 (mt, 2H, -CH< en 3a et 1H du -CH₂- en 3); 3,9 à 4,05 (mt, 2H, -CH< en 7a et l'autre H du -CH₂- en 3); 7,1 à 7,7 (mt, 10H aromatiques).

### Exemple 4

A une solution de 11,66 g de diphényl-7,7 tert-butoxycarbonyl-2 perhydroisoindolone-4-(3aS,7aS) dans 70 cm³ de tétrahydrofuranne sec, on ajoute goutte à goutte à température ambiante sous agitation une suspension de 10,78 g de bromure de phénylmagnésium dans 65 cm³ d'oxyde de diéthyle. Le mélange réactionnel est agité à température ambiante pendant 24 heures, porté au reflux pendant 5 heures, puis traité par 250 cm³ d'une solution aqueuse saturée en chlorure d'ammonium, dilué par 200 cm³ d'acétate d'éthyle, lavé par 200 cm³ d'eau (deux fois), puis par 200 cm³ d' une solution aqueuse saturée en chlorure de sodium, séché sur sulfate de magnésium, filtré et concentré à sec sous pression réduite (2,7 kPa). Le résidu est chromatographié sur colonne de gel de silice Merck (granulométrie 0,04-0,06 mm, diamètre 5,3 cm, hauteur 31,5 cm), en éluant sous une pression de 0,5 bar d'azote par un mélange de cyclohexane et d'acétate d'éthyle (90/10 en volumes) et en recueillant des fractions de 100 cm³. Les fractions 23 à 48 sont réunies et concentrées à sec sous pression réduite (2,7 kPa). On obtient 5,75 g de triphényl-4,7,7 tert-butoxycarbonyl-2 perhydroisoindolol-4-(3aS,4S,7aS) sous forme d'une meringue jaune pâle.

Spectre RMN du proton (DMSO d₆): 1,37 (s, 9H, -C(CH₃)₃); 1,65 (mt, 2H, -CH₂- en 5); 2,28 (d large, J=14, 1H, H équatorial du -CH₂- en 6); 2,65 (t, J=9, 1H du -CH₂- en 1); 2,85 (mt, 1H, -CH< en 3a); 3,05 (td, J=14 et 3,5; 1H, H axial du -CH₂- en 6); 3,25 (mt, 2H, l'autre H du -CH₂- en 1 et 1H du -CH₂- en 3); 3,4 (d, J=11, 1H, l'autre Hdu - CH₂- en 3); 3,5 (mt, 1H, -CH< en 7a); 4,4 (s, 1H, OH); 7,1 à 7,6 (mt, 15H aromatiques).

Spectre infra-rouge (KBr), bandes caractéristiques (cm⁻¹): 3425, 3100-3000, 3000-2850, 1680, 1600, 1580, 1495, 1475, 1410, 1365, 1170, 750, 700.

A une solution de 6,8 g de triphényl-4,7,7 tert-butoxycarbonyl-2 perhydroisoindolol-4-(3aS,4S,7aS) dans 60 cm³ de dioxanne on ajoute à température ambiante une solution de 115 cm³ de dioxanne chlorhydrique 6,3 N. Le mélange réactionnel est agité 2 heure à cette température, puis concentré à sec sous pression réduite (2,7 kPa). Le résidu est lavé par de l'acétonitrile, essoré puis séché. On obtient 4 g de chlorhydrate de triphényl-4,7,7 perhydroisoindolol-4-(3aS,4S,7aS) sous la forme d'une meringue blanche.

Spectre RMN du proton (DMSO d₆): 1,51 (td, J=14, 1H, H axial du -CH₂-en 5); 1,72 (d large, J=14, 1H, H équatorial du -CH₂- en 5); 2,34 (d large, J=14, 1H, H équatorial du -CH₂- en 6); 2,42 (td, J=10, 1H du -CH₂- en 1); 2,87 (td, J=14, 1H, H axial du -CH₂- en 6); 2,94 (mt, 1H, -CH< en 3a); 3,05 à 3,25 (mt, 3H, l'autre H du -CH₂- en 1 et le -CH₂- en 3); 3,57 (mt, 1H, -CH< en 7a); 5,67 (mf, 1H, OH); 7,1 à 7,6 (mt, 15H aromatiques); 8,9 (2mf, 1H chacun, NH₂⁺)

Spectre infra-rouge (KBr), bandes caractéristiques (cm⁻¹): 3500-3250, 3100-3000, 3000-2825, 2800-2250, 1600, 1580, 1495, 1445, 1075, 750, 700.

### Exemple 5

A une suspension de 26,4 g d'acétoxy-5 diphényl-7,7 (méthoxy-2 phényl)-4 tert-butoxycarbonyl-2 perhydroisoindolone-4-(3aRS,5RS,7aRs) et de 43,3 g de chlorure de cérium anhydre dans 265 cm³ de tétrahydrofuranne sec, on ajoute goutte à goutte à température ambiante sous agitation une suspension de 30,9 g de bromure de méthoxy-2 phénylmagnésium dans 170 cm³ de tétrahydrofuranne sec. Le mélange réactionnel est agité à température ambiante pendant 24 heures, traitée par 400 cm³ d'une solution aqueuse saturée en chlorure d'ammonium, repris par 1000 cm³ d'acétate d'éthyle puis filtré sur célite. La phase organique est décantée, séchée sur sulfate de magnésium, filtrée et concentrée à sec sous pression réduite (2,7 kPa). Le résidu est chromatographié sur colonne de gel de silice Merck (granulométrie 0,04-0,06 mm, diamètre 7 cm, hauteur 55 cm), en éluant sous une pression de 0,5 bar d'azote par un mélange de cyclohexane et d'acétate d'éthyle (70/10 en volumes) et en recueillant des fractions de 250 cm³. Les fractions 10 à 19 sont réunies et concentrées à sec sous pression réduite (2,7 kPa). On obtient 18 g de diphényl-7,7 (méthoxy-2 phényl)-4 tert-butoxycarbonyl-2 perhydroisoindolediol-4,5-(3aRS,4RS,5RS,7aRS) sous forme de cristaux blancs fondant à 229°C.

A une solution de 5,15 g de diphényl-7,7 (méthoxy-2 phényl)-4 tert-butoxycarbonyl-2 perhydroisoindolediol-4,5-(3aRS,4RS,5RS,7aRS) dans 25 cm³ de dioxanne, on ajoute à température ambiante une solution de 25 cm³ de dioxanne chlorhydrique 6N. Le mélange réactionnel est agité 1 heure à cette température, puis concentré à sec sous pression réduite (2,7 kPa). Le résidu est lavé par 20 cm³ l'acétonitrile, essoré et séché. On obtient 4,5 g de chlorhydrate de diphényl-7,7 (méthoxy-2 phényl)-4 perhydroisoindolediol-4,5-(3aRS,4RS,5RS,7aRS) sous forme de cristaux blancs fondant à une température supérieure à 300°C.

L'acétoxy-5 diphényl-7,7 tert-butoxycarbonyl-2 perhydroisoindolone-4-(3aRS,5RS,7aRS) peut être préparé de la manière suivante :

A une suspension de 19 g de chlorhydrate d'acétoxy-5 diphényl-7,7 perhydroisoindolone-4-(3aRS,5RS,7aRS) dans 200 cm³ de dichlorométhane sec, on ajoute à une température voisine de 5°C, sous agitation, 46,9 cm³ de triéthylamine, 11,8 g de di-tert-butyl dicarbonate, puis 0,3 g de diméthylamino-4 pyridine. Le mélange réactionnel est agité à température ambiante pendant 24 heures puis lavé par une solution aqueuse saturée en bicarbonate de sodium. La phase organique est décantée, séchée sur sulfate de magnésium, filtrée et concentrée à sec sous pression réduite (2,7 kPa). Le résidu est cristallisé dans 120 cm³ d'oxyde de diisopropyle. On obtient 21 g d'acétoxy-5 diphényl-7,7 perhydroisoindolone-4-(3aRS,5RS,7aRS) sous forme de cristaux blancs fondant à 213°C.

Le chlorhydrate d'acétoxy-5 diphényl-7,7 perhydroisoindo-lone-4-(3aRS,5RS,7aRS) peut être préparé de la manière suivante :

A une solution de 51,2 g d'acétoxy-5 diphényl-7,7 vinyloxycarbonyl-2 perhydroisoindolinone-4-(3aRS,5RS,7aRS) dans 118 cm³ de dioxanne, on ajoute à température ambiante, une solution de 394 cm³ de dioxanne chlorhydrique 5,2 N. Le mélange réactionnel est agité 1 heure à cette température, puis concentré à sec sous pression réduite (2,7 kPa). Le résidu est recristallisé dans 200 cm³ d'éthanol bouillant. On obtient 13,4 g de chlorhydrate d'acétoxy-5 diphényl-7,7 perhydroisoindolone-4-(3aRS,5RS,7aRS) sous forme de cristaux blancs fondant à une température supérieure à 300°C.

L'acétoxy-5 diphényl-7,7 vinyloxycarbonyl-2 perhydroisoindolino-ne-4-(3aRS,5RS,7aRS) peut être préparé de la manière suivante :

A une solution de 58 g d'acétoxy-5 benzyl-2 diphényl-7,7 perhydroisoindolinone-4-(3aRS,5RS,7aRS) dans 580 cm³ de dichlorométhane sec, on ajoute à température ambiante sous agitation 13,6 cm³ de chloroformiate de vinyle. Le mélange réactionnel est porté à reflux du solvant pendant une heure, refroidi à température ambiante et concentré à sec sous pression réduite (2,7 kPa). Le résidu est cristallisé dans 400 cm³ d'un mélange d'oxyde de diisopropyle et d'éther de pétrole (50/50 en volumes). On obtient 51,4 g d'acétoxy-5 diphényl-7,7 vinyloxycarbonyl-2 perhydroisoindolone-4-(3aRS,5RS,7aRS) sous forme de cristaux jaunes fondant à 205-210°C.

L'acétoxy-5 benzyl-2 diphényl-7,7 perhydroisoindolone-4-(3aRS,5RS,7aRS) peut être préparé de la manière suivante :

A une solution de 86 g d'acétoxy-6 diphényl-4,4 cyclohexènone-2 et de 96 cm³ de N-butoxyméthyl N-triméthylsilylméthyl benzylamine dans 1000 cm³ de dichlorométhane, on ajoute 15 gouttes d'acide trifluoroacétique. Le mélange réactionnel est agité à température ambiante 15 heures puis on ajoute 2 g de carbonate de sodium et concentre à sec sous pression réduite (2,7 kPa). Le résidu est chromatographié sur colonne de gel de silice Merck (granulométrie 0,04-0,06 mm, diamétre 7 cm, hauteur 70 cm), en éluant sous une pression de 0,5 bar d'azote par un mélange de cyclohexane et d'acétate d'éthyle (20/80 en volumes) et en recueillant des fractions de 200 cm³. Les fractions 6 à 10 sont réunies et concentrées à sec sous pression réduite (2,7 kPa). On obtient 70 g d'acétoxy-5 benzyl-2 diphényl-7,7 perhydroisoindolone-4-(3aRS,5RS,7aRS) sous forme de miel (point de fusion inférieur à 40°C).

La N-butoxyméthyl N-triméthylsilylméthyl benzylamine peut être préparée selon la méthode de Y. TERAO et coll., Chem. Pharm. Bull., 33, 2762 (1985).

L'acétoxy-6 diphényl-4,4 cyclohexènone-2 peut être préparée selon la méthode décrite par W.OPPOLZER et coll., Helv. Chim. Acta, 59, 2012 (1976).

### Exemple 6

Le chlorhydrate de diphényl-7,7 fluoro-4 (méthoxy-2 phényl)-4 perhydroisoindole-(3aS,4S,7aS) peut être obtenu de la manière suivante :

A une solution de 2,07 g de diphényl-7,7 fluoro-4 (méthoxy-2 phényl)-4 tert-butoxycarbonyl-2 perhydroisoindole-(3aS,4S,7aS) dans 20 cm³ de dioxanne, on ajoute à température ambiante une solution de 20 cm³ de dioxanne chlorhydrique 6,3 N. Le mélange réactionnel est agité à cette température pendant 3 heures, puis concentré à sec sous pression réduite (2,7 kPa). Le résidu est cristallisé dans 40 cm³ d'éthanol absolu, les cristaux sont essorés puis séchés. On obtient 1 g de chlorhydrate de diphényl-7,7 fluoro-4 (méthoxy-2 phényl)-4 perhydroisoindole-(3aS,4S,7aS) sous la forme de cristaux blancs fondant à 270°C.

Le diphényl-7,7 fluoro-4 (méthoxy-2 phényl)-4 tert-butoxycarbonyl-2 perhydroisoindole-(3aS,4S,7aS) peut être obtenu de la manière suivante :

A une solution refroidie à 0°C de 6,48 g de diphényl-7,7 (méthoxy-2 phényl)-4 tert-butoxycarbonyl-2 perhydroisoindolol-4-(3a5,4S,7aS) dans 70 cm³ de dichlorométhane sec, on ajoute goutte à goutte une solution de 4,3 cm³ trifluorure de diéthylaminosoufre dans 20 cm³ de dichlorométhane sec. Le mélange réactionnel est agité 3 heures à cette température, lavé par 100 cm³ d'une solution aqueuse saturée en hydrogénocarbonate de sodium, puis par 100 cm³ d'une solution aqueuse saturée en chlorure de sodium, séché sur sulfate de magnésium et concentré à sec sous pression réduite (2,7 kPa). Le résidu est chromatographié sur colonne de gel de silice Merck (granulométrie 0,04-0,06 mm, diamètre 4,8 cm, hauteur 26 cm), en éluant sous une pression de 0,5 bar d'azote par un mélange de cyclohexane et d'acétate d'éthyle (95/5 en volumes) et en recueillant des fractions de 50 cm³. Les fractions 34 à 63 sont réunies et concentrées à sec sous pression réduite (2,7 kPa). On obtient 2,2 g de diphényl-7,7 fluoro-4 (méthoxy-2 phényl)-4 tert-butoxycarbonyl-2 perhydroisoindole-(3aS,4S,7aS) sous la forme d'une meringue blanche.

### Exemple 7

Le chlorhydrate de diphényl-4,4 (méthoxy-2 phényl)-7 hexahydro-2,3,3a,4,5,7a isoindole-(3aS,7aR) peut être obtenu de la manière suivante :

Une solution de 8,56 g de diphényl-7,7 (méthoxy-2 phényl)-4 tert-butoxycarbonyl-2 perhydroisoindolol-4-(3aS,4S,7aS) dans 53 cm³ d'acide acétique et 30 cm³ d'acide chlorhydrique 12 N est chauffée à 95°C pendant 45 minutes puis refroidie à température ambiante et concentrée à sec sous pression réduite (2,7 kPa). Le résidu est recristallisé dans 20 cm³ d'acétonitrile. On obtient 5,2 g de chlorhydrate de diphényl-4,4 (méthoxy-2 phényl)-7 hexahydro-2,3,3a,4,5,7a isoindole-(3aS,7aR) sous forme de cristaux blancs fondant à une température supérieure à 300°C et utilisé à l'état brut dans les synthèses ultérieures.

### Exemple 8

A une suspension de 84,4 g de bromure de méthoxy-2 phénylmagnésium dans 1000 cm³ de tétrahydrofurane, on ajoute goutte à goutte, à température ambiante, sous agitation, une solution de 22 g d'acétoxy-5 benzyl-2 diphényl-7,7 perhydroisoindolone-4-(3aRS,5RS,7aRS) dans 220 cm³ de tétrahydrofurane. Le mélange réactionnel est agité à température ambiante pendant 18 heures, traité par 200 cm³ d'une solution aqueuse saturée en chlorure d'ammonium, repris par 200 cm³ d'oxyde d'éthyle et 200 g de glace. La phase organique est décantée, séchée sur sulfate de magnésium, filtrée et concentrée à sec sous pression réduite (2,7 kPa). Le résidu est cristallisé dans 250 cm³ d'éther de pétrole puis recristallisé dans 200 cm³ de méthanol; les cristaux sont lavés par 200 cm³ d'oxyde d'isopropyle. On obtient 16,4 g de benzyl-2 diphényl-7,7 (méthoxy-2 phényl)-4 perhydroisoindole-diol-4,5-(3aRS,4RS,5RS,7aRS) fondant à 236°C.

### Exemple 9

A 13 g de ditoluoyl-1,4-L-tartrate de diphényl-7,7 (méthoxy-2 phényl)-4 perhydroisoindolediol-4,5-(3aS,4S,5S,7aS) en solution dans 260 cm³ de méthanol, on ajoute 520 cm³ d'eau et 70 cm³ de soude aqueuse 1N; le mélange réactionnel est agité à température ambiante jusqu'à disparition du produit de départ. Les cristaux formés sont filtrés, essorés et séchés. On obtient 7,6 g de diphényl-7,7 (méthoxy-2 phényl)-4 perhydroisoindolediol-4,5-(3aS,4S,5S,7aS) fondant à 235°C.

Le ditoluoyl-1,4-L-tartrate de diphényl-7,7 (méthoxy-2 phényl)-4 perhydroisoindolediol-4,5-(3aS,4S,5S,7aS) peut être préparé de la manière suivante:

A une solution de 30 g de diphényl-7,7 (méthoxy-2 phényl)-4 perhydroisoindolediol-4,5-(3aRS,4RS,5RS,7aRS) dans 500 cm³ de méthanol, on ajoute, sous agitation, 29,2 g d'acide (-) ditoluoyl-1,4-L-tartrique. Après dissolution totale, le mélange réactionnel est concentré à sec sous pression réduite (2,7 kPa); la meringue obtenue est cristallisée dans 500 cm³ d'oxyde d'éthyle. Les cristaux obtenus sont recristallisés à pouvoir rotatoire constant dans un mélange d'éthanol et d'eau (60/40 en volumes). On obtient 13,3 g de ditoluoyl-1,4-L-tartrate de diphényl-7,7 (méthoxy-2 phényl)-4 perhydroisoindolediol-4,5-(3aS,4S,5S,7aS) fondant à 240°C.

Le diphényl-7,7 (méthoxy-2 phényl)-4 perhydroisoindolediol-4,5-(3aRS,4RS,5RS,7aRS) peut être préparé de la manière suivante:

Un mélange de 2 g de benzyl-2 diphényl-7,7 (méthoxy-2 phényl)-4 perhydroisoindolediol-4,5(3aRS,4RS,5RS,7aRS) et de 50 cm³ d'éthanol est chauffé à 65°C sous agitation; on ajoute 0,65 g d'hydroxyde de palladium sur charbon à 20% puis le mélange réactionnel est hydrogéné, sous agitation, à une température de 65°C et sous pression atmosphérique. Aprés 1 heure de réaction, le volume théorique d'hydrogène a été absorbé; le mélange réactionnel est filtré, puis concentré à sec sous pression réduite (2,7 kPa). Le résidu est cristallisé dans 10 cm³ d'oxyde d'isopropyle. On obtient 1,45 g de diphényl-7,7 (méthoxy-2 phényl)-4 perhydroisoindolediol-4,5-(3aRS, 4RS,5RS,7aRS) fondant à 230°C.

### Exemple d'utilisation 1

A une suspension de 0,8 g de chlorhydrate de diphényl-7,7 (méthoxy-2 phényl)-4 perhydroisoindolol-4-(3aS,4S,7aS) dans 60 cm³ de dichlorométhane sec on ajoute 0,025 g d'hydroxy-1 benzotriazole, 0,38 g d'acide (méthoxy-2 phényl)-2 propionique-(S), 0,32 cm³ de diisopropyléthylamine, puis on refroidit cette solution à +5°C et on ajoute rapidement une suspension de 0,43 g de chlorhydrate de (diméthylamino-3 propyl)-1 éthyl-3 carbodiimide dans 10 cm³ de dichlorométhane sec. Le mélange réactionnel est agité 2 heures à +5°C, 2 heures à température ambiante, lavé par 20 cm³ d'eau, puis lavé par 20 cm³ d'une solution aqueuse saturée en chlorure de sodium (deux fois), séché sur sulfate de magnésium, et concentré à sec sous pression réduite (2,7 kPa). Le résidu est chromatographié sur colonne de gel de silice Merck (granulométrie 0,04-0,06 mm, diamètre 2,8 cm, hauteur 20 cm), en éluant sous une pression de 0,5 bar d'azote par un mélange de cyclohexane et d'acétate d'éthyle (80/20 en volumes) et en recueillant des fractions de 25 cm^{3.} Les fractions 9 à 15 sont réunies puis concentrées à sec sous pression réduite (2,7 kPa). Le résidu est cristallisé dans un mélange d'acétonitrile et d'oxyde de diisopropyle. On obtient 0,17 g de diphényl-7,7 (méthoxy-2 phényl)-4 [(méthoxy-2 phényl)-2 propionyl-(S)]-2 perhydroisoindolol-4-(3aS,4S,7aS) sous la forme de cristaux blancs fondant à 244°C.

### Exemple d'utilisation 2

En opérant selon le mode opératoire de l'exemple d'utilisation 22 ci-après à partir de 0,68 g de chlorhydrate de diphényl-7,7 (méthyl-2 phényl)-4 perhydroisoindolol-4-(3aS,4S,7aS) et de 0,28 cm³ de chlorure de phénylacétyle on obtient 0,4 g de diphényl-7,7 (méthyl-2 phényl)-4 phénylacétyl-2 perhydroisoindolol-4-(3aS,4S,7aS) sous la forme de cristaux blancs fondant à 208°C.

### Exemple d'utilisation 3

A une suspension de 1,1 g de chlorhydrate de diphényl-7,7 (méthoxy-2 phényl)-4 perhydroisoindolol-4-(3aRS,4RS,7aRS) dans 25 cm³ de dichlorométhane sec on ajoute à température ambiante 0,35 cm³ de triéthylamine, puis 0,33 cm³ de chlorure de phénylacétyle. Le mélange réactionnel est agité 24 heures à cette température, dilué par 200 cm³ de dichlorométhane, lavé par 100 cm³ d'une solution saturée en hydrogénocarbonate de sodium, par 100 cm³ d'eau (deux fois), puis par une solution aqueuse saturée en chlorure de sodium, séché sur sulfate de magnésium, filtré et concentré à sec sous pression réduite (2,7 kPa).Le résidu est chromatographié sur colonne de gel de silice Merck (granulométrie 0,04-0,06 mm, diamètre 2 cm, hauteur 22 cm), en éluant sous une pression de 0,5 bar d'azote par un mélange de cyclohexane et d'acétate d'éthyle (55/45 en volumes) et en recueillant des fractions de 20 cm³. Les fractions 11 à 18 sont réunies et concentrées à sec sous pression réduite (2,7 kPa). Le résidu obtenu est cristallisé dans 70 cm³ d'acétonitrile, les cristaux sont essorés et séchés. On obtient 0,5 g de diphényl-7,7 (méthoxy-2 phényl)-4 phénylacétyl-2 perhydroisoindolol-4-(3aRS,4RS,7aRS) sous la forme d'un solide blanc.

Spectre RMN du proton (DMSO d₆): 1,5 (dmt, J=14, 1H, 1H équatorial du -CH₂- en 5); 2,26 (dmt, J=14, 1H, H équatorial du -CH₂- en 6); 2,31 (td, J=14 et 3, 1H, H axial du -CH₂- en 5); 2,85 (mt, 1H,-CH<en 3a); 3,02 (td, J=14 et 2,5, 1H, H axial du -CH₂- en 6); 3,2 à 3,6 (mf, -CH₂- et CH<); 3,44 (s, 3H, -OCH₃); 6,8 à 7,6 (mt, 19 H aromatiques).

Spectre infra-rouge (KBr), bandes caractéristiques (cm⁻¹): 3100-3000, 3000-2850, 2840, 1640, 1600, 1580, 1495, 1450, 1245, 1030,750, 720, 700.

### Exemple d'utilisation 4

A une suspension de 0,8 g de chlorhydrate de diphényl-7,7 (méthoxy-2 phényl)-4 perhydroisoindolol-4-(3aS,4S,7aS) dans 80 cm³ de dichlorométhane sec on ajoute 0,025 g d'hydroxy-1 benzotriazole, 0,27 cm³ d'acide phényl-2 propanoïque-(S), 0,32 cm³ de diisopropyléthylamine, puis on refroidit cette solution à +5°C et on ajoute rapidement une suspension de 0,43 g de chlorhydrate de (diméthylamino-3 propyl)-1 éthyl-3 carbodiimide dans 10 cm³ de dichlorométhane sec. Le mélange réactionnel est agité 2 heures à +5°C, 18 heures à température ambiante, lavé par 20 cm³ d'eau, puis lavé par 20 cm³ d'une solution aqueuse saturée en chlorure de sodium (deux fois), séché sur sulfate de magnésium, et concentré à sec sous pression réduite (2,7 kPa). Le résidu est chromatographié sur colonne de gel de silice Merck (granulométrie 0,04-0,06 mm, diamètre 2,8 cm, hauteur 20 cm), en éluant sous une pression de 0,5 bar d'azote par un mélange de cyclohexane et d'acétate d'éthyle (65/35 en volumes) et en recueillant des fractions de 25 cm^{3.} Les fractions 6 à 12 sont réunies puis concentrés à sec sous pression réduite (2,7 kPa). Le rédidu est rituré dans de l'oxyde de diisopopyle. On obtient 0,53 g de diphényl-7,7 (méthoxy-2 phényl)-4 [phényl-2 propionyl-(S)]-2 perhydroisoindole-4(3aS,4S,7aS) sous la forme de cristaux blancs fondant avec décomposition à 128°C.

### Exemple d'utilisation 5

A une suspension de 0,75 g de chlorhydrate de diphényl-7,7 (méthoxy-2 phényl)-4 perhydroisoindolol-4-(3aS,4S,7aS) dans 60 cm³ de dichlorométhane sec on ajoute 0,024 g d'hydroxy-1 benzotriazole, 0,33 g d'acide acétyloxy-2 phényl-2 acétique-(S), puis on refroidit cette solution à +5°C et on ajoute rapidement une suspension de 0,4 g de chlorhydrate de (diméthylamino-3 propyl)-1 éthyl-3 carbodiimide dans 20 cm³ de dichlorométhane sec, puis une solution de 0,63 cm³ de diisopropyléthylamine dans 20 cm³ de dichlorométhane sec. Le mélange réactionnel est agité 2 heures à +5°C, 24 heures à température ambiante, dilué par 120 cm³ de dichlorométhane, lavé par 100 cm³ d'eau, puis par 100 cm³ d'une solution aqueuse saturée en chlorure de sodium, séché sur sulfate de magnésium et concentré à sec sous pression réduite (2,7 kPa). Le résidu est chromatographié sur colonne de gel de silice Merck (granulométrie 0,04-0,06 mm, diamètre 2,5 cm, hauteur 39 cm), en éluant sous une pression de 0,5 bar d'azote par un mélange de cyclohexane et d'acétate d'éthyle (50/50 en volumes) et en recueillant des fractions de 60 cm^{3.} Les fractions 6 à 10 sont réunies puis concentrées à sec sous pression réduite (2,7 kPa). On obtient 0,96 g de diphényl-7,7 (méthoxy-2 phényl)-4 [acétyloxy-2 phényl-2 acétyl-(S)]-2 perhydroisoindolol-4-(3aS,4S,7aS) sous la forme d'une meringue blanche.

Spectre RMN du proton (DMSO d₆): A température ambiante, on observe le mélange des deux rotamères): 1,25 (dmt, J=14, H équatorial du -CH₂- en 5); 1,4 (dmt, J=14, H équatorial du -CH₂- en 5); 2,01 (s, 3H, -OCOCH₃); 2,27 (mt, 2H, H du -CH₂- en 5 et 1H du -CH₂- en 6); 2,65 à 3,6 (mt,-CH₂- et -CH<); 3,22 (s, 3H,-OCH₃); 4,38 (s, OH d'un rotamère); 4,86 (s, OH de l'autre rotamère); 5,66 (s, -CO-CH-O d'un rotamère); 5,88 (s, -CO-CH-O de l'autre rotamère); 6,6 à 7,6 (mt, 19H aromatiques).

Spectre infra-rouge (KBr), bandes caractéristiques (cm⁻¹): 3400, 3100-3000, 3000-2850, 2830, 1740, 1660, 1600, 1580, 1495, 1450, 1235, 1050, 755, 700.

A une solution de 0,8 g de diphényl-7,7 (méthoxy-2 phényl)-4 [acétyloxy-2 phényl-2 acétyl-(S)]-2 perhydroisoindolol-4-(3aS,4S,7aS) dans 30 cm³ d'éthanol on ajoute 1,4 cm³ d'une solution aqueuse de soude 1N, puis 10 cm³ d'eau. Le mélange réactionnel est porté au reflux pendant 1 heure, concentré à sec sous pression réduite (2,7 kPa), repris par 50 cm³ d'eau, puis 1,5 cm³ de solution aqueuse d'acide chlorhydrique 1N, et extrait par 40 cm³ d'acétate d'éthyle (3 fois). Les phases organiques sont réunies, lavées par 50 cm³ d'eau, séchées sur sulfate de magnésium et concentrées à sec sous pression réduite (2,7 kPa). Le résidu est chromatographié sur colonne de gel de silice Merck (granulométrie 0,04-0,06 mm, diamètre 2,5 cm, hauteur 27 cm), en éluant sous une pression de 0,4 bar d'azote par un mélange de cyclohexane et d'acétate d'éthyle (50/50 en volumes) et en recueillant des fractions de 30 cm³. Les fractions 2 à 5 sont réunies et concentrées à sec sous pression réduite (2,7 kPa). Le résidu est trituré dans de l'oxyde d'isopropyle. On obtient 0,6 g de diphényl-7,7 (méthoxy-2 phényl)-4 [hydroxy-2 phényl-2 acétyl-(S)]-2 perhydroisoindolol-4-(3aS,4S,7aS) sous la forme de cristaux blancs fondant à 256°C.

### Exemple d'utilisation 6

En opérant selon l'exemple d'utilisation 4 à partir de 2,25 g de chlorhydrate de diphényl-7,7 (méthoxy-2 phényl)-4 perhydroisoindolol-4-(3aS,4S,7aS) et de 1,25 g d'acide tert-butoxycarbonylamino-2 phényl-2 acétique-(S) on obtient 0,35 g de diphényl-7,7 (méthoxy-2 phényl)-4 [tert-butoxycarbonylamino-2 phényl-2 acétyl-(S)]-2 perhydroisoindolol-4-(3aS,4S,7aS) sous la forme d'une meringue blanche.

Spectre RMN du proton (DMSO d₆): A température ambiante, on observe le mélange des rotamères: 1,3 et 1,4 (2s, -C(CH₃)₃); 1,3 à 1,7 (mt, 1H du -CH₂- en 5); 2,15 à 2,45 (mt, 2H, l'autre H du -CH₂- en 5 et un H du -CH₂-en 6); 2,7 à 3,7 (mt, -CH₂- et -CH<); 3,33 et 3,4 (2s, -OCH₃); 4,84 et 5,11 (2s, 1H du NCO-CH-N des deux isomères); 6,6 à 7,7 (mt, 19H aromatiques).

Spectre infra-rouge (KBr), bandes caractéristiques (cm⁻¹) : 3580, 3550-3450, 3420, 3100-3000, 3000-2850, 2835, 1710, 1645, 1600, 1580, 1490, 1455, 1395, 1370, 1240, 1170, 1030.

En opérant selon l'exemple 1 à partir de 0,62 g de diphényl-7,7 (méthoxy-2 phényl)-4 [tert-butoxycarbonylamino-2 phényl-2 acétyl-(S)]-2 perhydroisoindolol-4-(3aS,4S,7aS) on obtient 0,54 g de chlorhydrate de diphényl-7,7 (méthoxy-2 phényl)-4 [amino-2 phényl-2 acétyl-(S)]-2 perhydroisoindolol-4-(3aS,4S,7aS) sous la forme d'une meringue blanche.

Spectre RMN du proton (DMSO d₆): On observe le mélange de diastéréoisomères: 1,34 et 1,53 (2dmt, J=14, 1H en totalité, H équatorial du -CH₂- en 5 pour les deux isomères); 2,31 (mt, 2H, l'autre H du -CH₂- en 5 et 1H du -CH₂- en 6); 2,8 à 3,7 (mt, -CH₂- et -CH<); 3,36 et 3,42 (2s, 3H, -OCH₃); 4,76 et 4,95 (2s, 1H, NCO-CH-N des deux isomères); 6,6 à 7,7 (mt, 19H aromatiques).

Spectre infra-rouge (KBr), bandes caractéristiques (cm⁻¹) : 3425, 3100-3000, 3000-2850, 2830, 3150-2500, 1660, 1600, 1585, 1495, 1450, 1235, 1030, 755, 700.

### Exemple d'utilisation 7

A une solution de 0,28 g d'acide (méthoxy-2 phényl)-acétique dans 20 cm³ de dichlorométhane sec on ajoute 0,28 g de carbonyldiimidazole. On agite à température ambiante pendant 1 heure, puis on ajoute successivement une suspension de 0,7 g de chlorhydrate de triphényl-4,7,7 perhydroisoindolol-4-(3aS,4S,7aS) dans 20 cm³ de dichlorométhane sec, puis 0,48 cm³ de triéthylamine. Le mélange réactionnel est agité 24 heures à température ambiante, dilué par 100 cm³ de dichlorométhane, lavé par 100 cm³ d'eau (deux fois), puis par 100 cm³ d'une solution aqueuse saturée en chlorure de sodium, séché sur sulfate de magnésium, et concentré à sec sous pression réduite (2,7 kPa). Le résidu est chromatographié sur colonne de gel de silice Merck (granulométrie 0,04-0,06 mm, diamètre 2 cm, hauteur 16 cm), en éluant sous une pression de 0,5 bar d'azote par un mélange de cyclohexane et d'acétate d'éthyle (55/45 en volumes) et en recueillant des fractions de 20 cm^{3.} Les fractions 7 à 8 sont réunies puis concentrées à sec sous pression réduite (2,7 kPa). Le résidu est cristallisé dans 16 cm³ d'acétonitrile. Les cristaux sont essorés et séchés. On obtient 0,3 g de triphényl-4,7,7 (méthoxy-2 phényl)-acétyl-2 perhydroisoindolol-4-(3aS,4S,7aS) sous la forme de cristaux blancs fondant à 236°C.

### Exemple d'utilisation 8

En opérant selon l'exemple d'utilisation 4 à partir de 1,13 g de chlorhydrate de diphényl-7,7 (méthoxy-2 phényl)-4 perhydroisoindolol-4-(3aS,4S,7aS) et de 0,42 g d'acide (méthoxy-2 phényl)-acétique on obtient 0,61 g de diphényl-7,7 (méthoxy-2 phényl)-4 [(méthoxy-2 phényl)-acétyl]-2 perhydroisoindolol-4-(3aS,4S,7aS) sous la forme de cristaux blancs fondant à 204°C.

### Exemple d'utilisation 9

En opérant selon l'exemple d'utilisation 4 à partir de 0,75 g de chlorhydrate de diphényl-7,7 (méthoxy-2 phényl)-4 perhydroisoindolol-4-(3aS,4S,7aS) et de 0,28 g d'acide (méthoxy-3 phényl)-acétique on obtient 0,54 g de diphényl-7,7 (méthoxy-2 phényl)-4 [(méthoxy-3 phényl)-acétyl]-2 perhydroisoindolol-4-(3aS,4S,7aS) sous la forme de cristaux blancs fondant à 185°C.

### Exemple d'utilisation 10

En opérant selon l'exemple d'utilisation 4 à partir de 0,75 g de chlorhydrate de diphényl-7,7 (méthoxy-2 phényl)-4 perhydroisoindolol-4-(3aS,4S,7aS) et de 0,28 g d'acide (méthoxy-4 phényl)-acétique on obtient 0,61 g de diphényl-7,7 (méthoxy-2 phényl)-4 [(méthoxy-4 phényl)-acétyl]-2 perhydroisoindolol-4-(3aS,4S,7aS) sous la forme de cristaux blancs fondant à 211°C.

### Exemple d'utilisation 11

En opérant selon l'exemple d'utilisation 4 à partir de 0,8 g de chlorhydrate de diphényl-7,7 (méthoxy-2 phényl)-4 perhydroisoindolol-4-(3aS,4S,7aS) et de 0,38 g d'acide naphtyl-1 acétique on obtient 1,16 g de diphényl-7,7 (méthoxy-2 phényl)-4 (naphtyl-1 acétyl)-2 perhydroisoindolol-4-(3aS,4S,7aS) sous la forme d'un solide blanc fondant à 225°C.

### Exemple d'utilisation 12

En opérant selon l'exemple d'utilisation 4 à partir de 0,8 g de chlorhydrate de diphényl-7,7 (méthoxy-2 phényl)-4 perhydroisoindolol-4-(3aS,4S,7aS) et de 0,34 g d'acide thiényl-3 acétique on obtient 0,53 g de diphényl-7,7 (méthoxy-2 phényl)-4 [(thiényl-3)-acétyl]-2 perhydroisoindolol-4-(3aS,4S,7aS) sous la forme de cristaux blancs fondant avec décomposition à 106°C.

### Exemple d'utilisation 13

A une solution de 0,44 g d'acide indolyl-3 acétique dans 20 cm³ de dichlorométhane sec on ajoute 0,41 g de cabonyldiimidazole. On agite à température ambiante pendant 1 heure, puis on ajoute successivement une suspension de 1,1 g de chlorhydrate de diphényl-7,7 (méthoxy-2 phényl)-4 perhydroisoindolol-4-(3aRS,4RS,7aRS) dans 25 cm³ de dichlorométhane sec, puis 0,7 cm³ de triéthylamine. Le mélange réactionnel est agité 24 heures à température ambiante, dilué par 100 cm³ de dichlorométhane, lavé par 100 cm³ d'eau (deux fois), puis par 100 cm³ d'une solution aqueuse saturée en chlorure de sodium, séché sur sulfate de magnésium, et concentré à sec sous pression réduite (2,7 kPa). Le résidu est chromatographié sur colonne de gel de silice Merck (granulométrie 0,04-0,06 mm, diamètre 2 cm, hauteur 22 cm), en éluant sous une pression de 0,5 bar d'azote par un mélange de cyclohexane et d'acétate d'éthyle (35/65 en volumes) et en recueillant des fractions de 20 cm^{3.} Les fractions 7 à 8 sont réunies puis concentrées à sec sous pression réduite (2,7 kPa). Le résidu est trituré dans de l'oxyde de diisopropyle. On obtient 0,17 g de diphényl-7,7 (indolyl-3 acétyl)-2 (méthoxy-2 phényl)-4 perhydroisoindolol-4-(3aRS,4RS,7aRS) sous la forme d'un solide blanc.

Spectre RMN du proton(DMSO d₆): 1,48 (d large, J=14,5,1H, H équatorial du -CH₂- en 5); 2,27 (d large, J=14,5, 1H, H équatorial du -CH₂- en 6); 2,32 (td, J=14,5 et 2, 1H, H axial du -CH₂- en 5); 3,02 (td, J=14,5 et 2, 1H, H axial du -CH₂- en 6); 2,88 et 3,2 à 3,7 (2mt, respectivement 1H et 5H, -CH₂- et -CH); 3,44 (s, 3H, -OCH₃); 3,52 (s, 2H, -N-COCH₂-); 6,8 à 7,6 (mt, 19H, aromatiques).

Spectre infra-rouge (KBr), bandes caractéristiques (cm⁻¹): 3425, 3125-3000, 3000-2850, 1625, 1585, 1490, 1460, 1235, 1030, 745, 700

### Exemple d'utilisation 14

A une suspension de 0,96 g de chlorhydrate de diphényl-7,7 (méthoxy-2 phényl)-4 perhydroisoindolol-4-(3aS,4S,7aS) dans 25 cm³ de dichlorométhane sec, on ajoute successivement à température ambiante sous agitation une solution de 0,43 g de chlorure d'(indolyl-3)-2 oxo-2 acétyle dans 20 cm³ de dichlorométhane sec, puis une solution de 0,6 cm³ de triéthylamine dans 5 cm³ de dichlorométhane sec. Le mélange réactionnel est agité 24 heures à cette température, puis dilué par 200 cm³ de dichlorométhane, lavé par 100 cm³ de solution aqueuse de soude 1N, par 50 cm³ d'une solution aqueuse saturée en chlorure de sodium, séché sur sulfate de magnésium et concentré à sec sous pression réduite (2,7 kPa). Le résidu est chromatographié sur colonne de gel de silice Merck (granulométrie 0,04-0,06 mm, diamètre 2,5 cm, hauteur 31 cm), en éluant sous une pression de 0,4 bar d'azote par un mélange de dichloro-1,2 éthane et de méthanol (70/30 en volumes) et en recueillant des fractions de 15 cm³. Les fractions 2 à 9 sont réunies et concentrées à sec sous pression réduite (2,7 kPa). On obtient 1,15 g de diphényl-7,7 [oxo-2 (indolyl-3)-2 acétyl]-2 (méthoxy-2 phényl)-4 perhydroisoindolol-4-(3aS,4S,7aS) sous la forme d'une meringue orange.

Spectre infra-rouge (KBr), bandes caractéristiques (cm⁻¹): 3400, 3250, 3100-3000, 3000-2850, 2835, 1650-1600, 1580, 1520, 1490, 1455, 1235, 1030, 755, 700.

A une solution de 1,1 g de diphényl-7,7 [oxo-2 (indolyl-3)-2 acétyl-(RS)]-2 (méthoxy-2 phényl)-4 perhydroisoindolol-4- (3aS,4S,7aS) dans 35 cm³ d'éthanol on ajoute, à température ambiante sous agitation, 0,38 g de borohydrure de sodium. Le mélange réactionnel est agité 4 heures à cette température, puis traité par 2 cm³ d'acide acétique et concentré à sec sous pression réduite (2,7 kPa). Le résidu est dissous dans 100 cm³ d'acétate d'éthyle, la phase organique est lavée par 50 cm³ d'une solution aqueuse de soude 0,1N, par 50 cm³ d'eau, puis par 50 cm³ d'une solution aqueuse saturée en chlorure de sodium, séchée sur sulfate de magnésium et concentrée à sec sous pression réduite (2,7 kPa). Le résidu est chromatographié sur colonne de gel de silice Merck (granulométrie 0,04-0,06 mm, diamètre 2,8 cm, hauteur 27 cm), en éluant sous une pression de 0,4 bar d'azote par un mélange de dichloro-1,2 éthane et de méthanol (96/4 en volumes) et en recueillant des fractions de 10 cm³. Les fractions 17 à 31 sont réunies et concentrées à sec sous pression réduite (2,7 kPa). Le résidu est trituré dans de l'oxyde de diisopropyle. On obtient 0,45 g de diphényl-7,7 [hydroxy-2 (indolyl-3)-2 acétyl-(RS)]-2 (méthoxy-2 phényl)-4 perhydroisoindolol-4-(3aS,4S,7aS) sous la forme d'une meringue blanche.

Spectre RMN du proton (DMSO d₆): A température ambiante, on observe un mélange d'isomères et de rotamères: 1,4 (mt, 1H, H équatorial du -CH₂- en 5); 2,3 (mt, 2H, H axial du -CH₂- en 5 et 1H du -CH₂- en 6); 2,5 à 3,8 (mt, -CH₂- et -CH<); 3,30-3,32-3,35 et 3,38 (4s, OCH₃ des différents isomères et rotamères); 5-5,12-5,24 et 5,28 (4s, 1H, N-CO -CH-O des différents isomères et rotamères); 6,5 à 7,8 (mt aromatiques).

Spectre infra-rouge (KBr), bandes caractéristiques (cm⁻¹): 3420, 3125-3000, 3000-2850, 2830, 1630, 1600, 1580, 1495, 1450, 1235, 1030, 755, 745, 700.

### Exemple d'utilisation 15

En opérant selon le mode opératoire de l'exemple d'utilisation 4 à partir de 0,8 g de chlorhydrate de diphényl-7,7 (méthoxy-2 phényl)-4 perhydroisoindolol-4-(3aS,4S,7aS) et de 0,39 g d'acide (fluoro-5 indolyl-3)-acétique on obtient 0,36 g de diphényl-7,7 [(fluoro-5 indolyl-3)-acétyl]-2 (méthoxy-2 phényl)-4 perhydroisoindo-lol-4-(3aS,4S,7aS) sous la forme de cristaux blancs fondant avec décomposition à 170°C.

### Exemple d'utilisation 16

En opérant selon l'exemple d'utilisation 4 à partir de 0,8 g de chlorhydrate de diphényl-7,7 (méthoxy-2 phényl)-4 perhydroisoindolol-4-(3aS,4S,7aS) et de 0,41 g d'acide (méthoxy-5 indolyl-3)-acétique on obtient 0,66 g de diphényl-7,7 [(méthoxy-5 indolyl-3)- acétyl]-2 (méthoxy-2 phényl)-4 perhydroisoindolol-4-(3aS,4S,7aS) sous la forme d'une meringue beige.

Spectre RMN du proton (DMSO d₆): 1,5 (d large, J=14, 1H, H équatorial du -CH₂- en 5); 2,29 (d large, J=14, 1H, H équatorial du -CH₂- en 6); 2,35 (td, J=14 et 2,5; 1H axial du -CH₂- en 5); 3,04 (td, J=14 et 2,5; H axial du -CH₂- en 6); 2,8 à 3,9 (mt, -CH₂- et -CH<); 3,44 (s, -OCH₃); 3,75 (s, NCO-CH₂-); 3,89 (s, 3H, -OCH₃ de l'indole); 6,7 à 7,7 (mt, 18H aromatiques); 10,3 (mf, 1H, NH de l'indole).

Spectre infra-rouge (KBr), bandes caractéristiques (cm⁻¹): 3300-2200, 3125-3000, 3000-2850, 2830, 1625, 1600, 1580, 1485, 1450, 1230, 1215, 1025, 755, 700.

### Exemple d'utilisation 17

En opérant selon l'exemple d'utilisation 4 à partir de 0,75 g de chlorhydrate de diphényl-7,7 (méthoxy-2 phényl)-4 perhydroisoindolol-4-(3aS,4S,7aS) et de 0,32 g d'acide (méthyl-1 indolyl-3)-acétique on obtient 0,56 g de diphényl-7,7 (méthoxy-2 phényl)-4 [(méthyl-1 indolyl-3)-acétyl)-2 perhydroisoindolol-4-(3aS,4S,7aS) sous la forme d'une meringue beige.

Spectre RMN du proton (DMSO d₆): A température ambiante, on observe le mélange des deux rotamères): 1,42 (mt, 1H, 1H du -CH₂- en 5); 2,31 (mt, 2H, l'autre H du -CH₂- en 5 et 1H du -CH₂- en 6); 2,94 (mt, l'autre H du -CH₂- en 6); 2,7 à 3,6 (mt, -CH₂- et -CH<); 3,37 (s, 3H, -OCH₃); 3,45 et 3,5 (2s, 2H, -COCH₂Ar); 3,72 et 3,78 (2s, 3H, NCH₃); 6,8 à 7,7 (mt, 19H aromatiques).

Spectre infra-rouge (KBr), bandes caractéristiques (cm⁻¹): 3400, 3125-3000, 3000-2850, 2830, 1637, 1600, 1580, 1485, 1450, 1235, 1050, 750, 700.

### Exemple d'utilisation 18

A une solution de 4,5 g de chlorhydrate de diphényl-7,7 (méthoxy-2 phényl)-4 perhydroisoindolediol-4,5-(3aRS,4RS,5RS,7aRS) dans 100 cm³ de dichlorométhane, refroidie à 0°C, on ajoute 4,2 cm³ de triéthylamine puis 2,4 g du chlorure de l'acide (méthoxy-2 phényl)acétique dans 50 cm³ de dichlorométhane. On agite à température ambiante pendant 90 minutes; le mélange réactionnel est lavé par 2 fois 10 cm³ d'eau, séché sur sulfate de magnésium et concentré à sec sous pression réduite (2,7 kPa). Le solide cristallisé est repris par 100 cm³ d'oxyde de diisopropyle puis filtré, lavé par 50 cm³ d'une solution saturée de bicarbonate de sodium puis 50 cm³ d'oxyde de diisopropyle. On obtient 4,35 g de diphényl-7,7 (méthoxy-2 phényl)-4 (méthoxy-2 phényl) acétyl-2 perhydroisoindolediol-4,5-(3aRS,4RS,5RS,7aRS) sous forme d'un solide beige clair fondant à 278°C.

Le chlorure de l'acide (méthoxy-2 phényl)acétique est obtenu à partir d'un mélange de 2,2 g d'acide (méthoxy-2 phényl)acétique et de 20 cm³ de chlorure de thionyle, porté à reflux 30 minutes. Après concentration à sec sous pression réduite (2,7 kPa), on obtient 2,4 g d'une huile jaune utilisée à l'état brut dans les synthèses ultérieures.

### Exemple d'utilisation 19

En opérant selon l'exemple d'utilisation 18 à partir de 0,5 g chlorhydrate de diphényl-7,7 phényl-4 perhydroisoindolediol-4,5-(3aS,4S,5S,7aS) et de 0,39 g d'acide (méthoxy-2 phényl)-acétique, on obtient 0,4 g de diphényl-7,7 phényl-4 (méthoxy-2 phényl) acétyl-2 perhydroisoindoledio1-4,5-(3aS,4S,5S,7aS) sous forme d'un solide blanc fondant avec décomposition à 150°C.

### Exemple d'utilisation 20

En opérant selon l'exemple 1 à partir de 0,5 g chlorhydrate de diphényl-7,7 phényl-4 perhydroisoindolediol-4,5-(3aS,4S,5S,7aS) et de 0,25 g d'acide (méthoxy-2 phényl)-2 propionique-(S), on obtient 0,52 g de diphényl-7,7 phényl-4 [(méthoxy-2 phényl)-2 propionyl-(S)]-2 perhydroisoindoledio1-4,5-(3aS,4S,5S,7aS) sous forme d'un solide blanc fondant avec décomposition à 158°C.

### Exemple d'utilisation 21

A une solution de 0,9 g de chlorhydrate de diphényl-7,7 (méthoxy-2 phényl)-4 perhydroisoindolediol-4,5-(3aRS,4RS,5RS,7aRS), de 0,4 g d'acide indolyl-3 acétique et de 20 mg d'hydrate d' hydroxy-1-benzotriazole dans 90 cm³ de dichlorométhane, refroidie à 0°C, on ajoute 0,46 g de (diméthylamino-3 propyl)-1 éthyl-3 carbodiimide et 0,34 cm³ de diisopropylamine. On agite à température ambiante pendant 15 heures, acidifie par HCl 0,1 N puis reprend par une solution aqueuse saturée de chlorure de sodium. La phase organique est décantée, séchée sur sulfate de magnésium et concentrée à sec sous pression réduite (2,7 kPa). La meringue obtenue est recristallisée dans 10 cm³ d'acétonitrile bouillant. On obtient 0,85 g de diphényl-7,7 (méthoxy-2 phényl)-4 (indolyl-3) acétyl-2 perhydroisoindolediol-4,5-(3aRS,4RS,5RS,7aRS) sous forme d'un solide blanc fondant à 266°C.

### Exemple d'utilisation 22

A une suspension de 0,14 g de chlorhydrate de diphényl-7,7 fluoro-4 (méthoxy-2 phényl)-4 perhydroisoindole-(3aS,4S,7aS) dans 7 cm³ de dichlorométhane sec on ajoute à température ambiante 0,1 cm³ de triéthylamine, puis 0,04 cm³ de chlorure de phénylacétyle. Le mélange réactionnel est agité 5 heures à cette température, dilué par 100 cm³ de dichlorométhane, lavé par 40 cm³ d'une solution saturée en hydrogénocarbonate de sodium, puis par 40 cm³ d'eau, séché sur sulfate de magnésium et concentré à sec sous pression réduite (2,7 kPa). Le résidu obtenu est trituré dans de l'oxyde de diisopropyle. On obtient 0,1 g de diphényl-7,7 fluoro-4 (méthoxy-2 phényl)-4 phénylacétyl-2 perhydroisoindole-(3aS,4S,7aS) sous la forme d'une meringue blanche.

Spectre RMN du proton (DMSO D₆): A température ambiante, on observe un mélange de rotamères: 1,62 (mt, 1H, 1H du -CH₂- en 5); 2 à 3,8 (mt, -CH₂- et -CH<); 3,38 et 3,42 (2s, 3H, -OCH₃); 6,7 à 7,6 (mt, 19H, aromatiques)

Spectre infra-rouge (KBr), bandes caractéristiques (cm⁻¹): 3100-3000, 3000-2850, 2840, 1640, 1600, 1580, 1495, 1455, 1240, 1030, 755, 720, 700

### Exemple d'utilisation 23

A une solution de 1,5 g de chlorhydrate de diphényl-4,4 (méthoxy-2 phényl)-7 hexahydro-2,3,3a,4,5,7a isoindole-(3aS,7aR) dans 20 cm³ de dichlorométhane, refroidie à 0°C, on ajoute 1,5 cm³ de triéthylamine puis 1,6 g du chlorure de l'acide (méthoxy-2 phényl)acétique. On agite à température ambiante pendant 15 heures; le mélange réactionnel est lavé par 2 fois 40 cm³ d'eau, séché sur sulfate de magnésium et concentré à sec sous pression réduite (2,7 kPa). Le résidu est chromatographié sur colonne de gel de silice Merck (granulométrie 0,04-0,06 mm, diamètre 3,6 cm, hauteur 25 cm), en éluant sous une pression de 0,5 bar d'azote par un mélange de cyclohexane et d'acétate d'éthyle (75/25 en volumes) et en recueillant des fractions de 25 cm³. Les fractions 14 à 28 sont réunies et concentrées à sec sous pression réduite (2,7 kPa). La meringue jaune obtenue est recristallisée dans 10 cm³ de cyclohexane pour fournir 0,38 g de diphényl-4,4 (méthoxy-2 phényl)acétyl-2 (méthoxy-2 phényl)-7 hexahydro-2,3,3a,4,5,7a isoindole-(3aS,7aR) sous forme d'un solide beige fondant à 142°C.

### Exemple d'utilisation 24

En opérant selon l'exemple d'utilisation 4, mais à partir de 2 g de chlorhydrate de diphényl-7,7 (méthoxy-2 phényl)-4 perhydroisoindolol-4-(3aS,4S,7aS) et de 0,96 g d'acide indolyl-3 acétique on obtient 2,17 g de diphényl-7,7 (méthoxy-2 phényl)-4 [(indolyl-3)-acétyl]-2 perhydroisoindolol-4-(3aS,4S,7aS) sous la forme de cristaux blancs fondant à 142°C.

### Exemple d'utilisation 25

En opérant selon l'exemple d'utilisation 4, mais à partir de 0,73 g de chlorhydrate de diphényl-7,7 (méthoxy-2 phényl)-4 perhydroisoindolol-4-(3aS,4S,7aS) et de 0,5 g d'acide (benzyloxy-2 phényl)-2 propionique-(S) on obtient 0,73 g de diphényl-7,7 (méthoxy-2 phényl)-4 [(benzyloxy-2 phényl)-2 propionyl-(S)]-2 perhydroisoindolol-4-(3aS,4S,7aS) sous la forme d'une meringue blanche.

Spectre RMN du proton (DMSO d₆): A température ambiante, on observe le mélange des rotamères: 1,15 (2d, 3H du CH₃-CH); 3,35 (2s, 3H du OCH₃); 3,98 et 3,78 (2q, 1H du CH-CH₃); 4,2 (s, 1H du OH); 4,6 à 5 (2dd, 2H du CH₂O); 6,7 à 7,6 (m, 23H aromatiques).

A une solution de 0,73 g de diphényl-7,7 (méthoxy-2 phényl)-4 [(benzyloxy-2 phényl)-2 propionyl-(S)]-2 perhydroisoindolol-4-(3aS,4S,7aS) obtenus précédemment, dans 20 cm³ d'éthanol absolu, on ajoute 0,005 g de palladium sur charbon à 10% et on fait buller, à température ambiante de l'hydrogène dans le mélange réactionnel pendant 2 heures. Le mélange réactionnel est filtré sur célite, puis concentré à sec sous pression réduite (2,7 kPa). Le résidu est chromatographié sur colonne de gel de silice Merck (granulométrie 0,04-0,06 mm, diamètre 2,4 cm, hauteur 20 cm), en éluant sous une pression de 0,5 bar d'azote par un mélange de cyclohexane et d'acétate d'éthyle (80/20 en volumes) et en recueillant des fractions de 30 cm³. Les fractions 3 à 8 sont réunies et concentrées à sec sous pression réduite (2,7 kPa). On obtient 0,2 g de diphényl-7,7 (méthoxy-2 phényl)-4 [(hydroxy-2 phényl)-2 propionyl-(S)]-2 perhydroisoindolol-4-(3aS,4S,7aS) sous la forme de cristaux blancs fondant à 150°C avec décomposition.

L'acide (benzyloxy-2 phényl)-2 propionique-(S) peut être obtenu de la manière suivante :

Une solution de 1,07 g de N-[(benzyloxy-2 phényl)-2-(S) propionyl] camphor-2,10-sultame-(1R,2S) dans un mélange de 0,47 cm³ de solution aqueuse de soude à 30% et de 10 cm³ de tétrahydrofurane est agitée à température ambiante pendant 48 heures. Le mélange réactionnel est concentré à sec sous pression réduite (2,7 kPa), puis dilué par 20 cm³ d'eau distillée, la phase aqueuse est extraite par 25 cm³ de dichlorométhane, puis acidifiée par 3 cm³ de solution aqueuse d'acide chlorhydrique à 37%, et enfin extraite 3 fois par 25 cm³ de dichlorométhane. La phase organique est séchée sur sulfate de magnésium et concentrée à sec sous pression réduite (2,7 kPa). On obtient 0,5 g d'acide (benzyloxy-2 phényl)-2 propionique-(S) sous la forme d'une huile incolore.

Spectre RMN du proton (CDCl₃): 1,5 (d, 3H du CH₃-CH); 4,18 (q, 1H du CH-CH₃); 5,1 (A₂, 2H du OCH₂); 6,95 à 7,45 (m, 9H aromatiques).

Le N-[(benzyloxy-2 phényl)-2-(S) propionyl] camphor-2,10-sultame-(1R,2S) peut être préparé de la manière suivante :

A une solution de 4,1 g de N-[(benzyloxy-2 phényl)-acétyl] camphor-2,10-sultame-(1R,2S) dans 40 cm³ de tétrahydrofurane refroidie à-78°C on ajoute par fractions 1,62 g de tertiobutylate de potassium, puis on ajoute goutte à goutte à cette suspension une solution de 2,63 cm³ d'ioduré de méthyle dans 2 cm³ de tétrahydrofurane. Le mélange réactionnel est agité à -78°C pendant 18 heures, puis on ajoute 40 cm³ d'une solution aqueuse saturée en chlorure d'ammonium. Après retour à température ambiante, le mélange réactionnel est extrait par 80 cm³ d'acétate d'éthyle, la phase organique est lavée deux fois par 25 cm³ d'une solution aqueuse saturée en chlorure d'ammonium, séchée sur sulfate de magnésium et concentrée à sec sous pression réduite (2,7 kPa). Le résidu est chromatographié sur colonne de gel de silice Merck (granulométrie 0,04-0,06 mm, diamètre 4 cm, hauteur 39 cm), en éluant sous une pression de 0,3 bar d'azote par du dichlorométhane et en recueillant des fractions de 25 cm³. Les fractions 21 à 53 sont réunies et concentrées à sec sous pression réduite (2,7 kPa). Le résidu est cristallisé dans l'oxyde de diisopropyle. On obtient 1,1 g de N-[(benzyloxy-2 phényl)-2-(S) propionyl] camphor-2,10-sultame-(1R,2S) sous la forme de cristaux blancs fondant à 131°C.

Le N-[(benzyloxy-2 phényl)-acétyl] camphor-2,10-sultame-(1R,2S) peut être préparé de la manière suivante :

A une solution refroidie à +10°C de 3,23 g de camphor-2,10-sultame-(1R,2S) dans 16 cm³ de dichlorométhane sec, on ajoute goutte à goutte une solution de 0,74 g de soude dans 20 cm³ d'eau distillée, puis 0,03 cm³ d'Aliquat 336®. On ajoute ensuite goutte à goutte à +10°C une solution de 3,9 g de chlorure de (méthoxy-2 phényl)-acétyle dans 5 cm³ de dichlorométhane. Le mélange réactionnel est agité 1 heure à +10°C, décanté, la phase aqueuse est extraite par 80 cm³ de dichlorométhane, les phases organiques sont réunies, lavées par 80 cm³ d'eau distillée, séchées sur sulfate de magnésium et concentrées à sec sous pression réduite (2,7 kPa). Le résidu est cristallisé dans 15 cm³ d'oxyde de diisopropyle. On obtient 4,1 g de N-[(benzyloxy-2 phényl)-acétyl] camphor-2,10-sultame-(1R,2S) sous la forme de cristaux blancs fondant à 116°C.

### Exemple d'utilisation 26

En opérant selon l'exemple d'utilisation 4, mais à partir de 12,27 g de chlorhydrate de diphényl-7,7 (méthoxy-2 phényl)-4 perhydroisoindolol-4 et de 7,88 g d'acide (benzyloxy-2 phényl)-acétique on obtient 16,4 g de diphényl-7,7 (méthoxy-2 phényl)-4 [(benzyloxy-2 phényl) acétyl]-2 perhydroisoindolol-4-(3aS,4S,7aS) sous la forme d'une meringue blanche.

Spectre RMN du proton (DMSO d₆): A température ambiante, on observe le mélange des rotamères: 3,4 (s, 3H du OCH₃); 5 (s, 2H du -CH₂O); 6,85 à 7,5 (m, 23H aromatiques).

En opérant selon l'exemple d'utilisation 25 à partir de 16,4 g de diphényl-7,7 (méthoxy-2 phényl)-4 [(benzyloxy-2 phényl)-2 acétyl]-2 perhydroisoindolol-4-(3aS,4S,7aS) on obtient 11,4 g de diphényl-7,7 (méthoxy-2 phényl)-4 [(hydroxy-2 phényl)-2 acétyl]-2 perhydroisoindolol-4-(3aS,4S,7aS) sous la forme de cristaux blancs fondant à 190°C.

### Exemple d'utilisation 27

A une suspension de 5 g de chlorhydrate de diphényl-4,4 (méthoxy-2 phényl)-7 hexahydro-2,3,3a,4,5,7a-(1H)-isoindole-(3aS,7aR) dans 70 cm³ de dichlorométhane sec on ajoute 0,16 g d'hydroxy-1 benzotriazole, 2,59 g d'acide (méthoxy-2 phényl)-2 propionique-(S), 2,08 cm³ de diisopropyléthylamine, puis on refroidit cette solution à +5°C et on ajoute rapidement une suspension de 2,8 g de chlorhydrate de (diméthylamino-3 propyl)-1 éthyl-3 carbodiimide dans 10 cm³ de dichlorométhane sec. Le mélange réactionnel est agité 2 heures à +5°C, puis 24 heures à température ambiante, lavé par 20 cm³ d'eau, puis par 20 cm³ d'une solution aqueuse saturée en chlorure de sodium (deux fois), séché sur sulfate de magnésium, et concentré à sec sous pression réduite (2,7 kPa). Le résidu est chromatographié sur colonne de gel de silice Merck (granulométrie 0,04-0,06 mm, diamètre 4 cm, hauteur 20 cm), en éluant sous une pression de 0,5 bar d'azote par un mélange de cyclohexane et d'acétate d'éthyle (80/20 en volumes) et en recueillant des fractions de 50 cm³. Les fractions 2 à 8 sont réunies et concentrées à sec sous pression réduite (2,7 kPa). Le résidu est trituré dans l'oxyde de diisopropyle. On obtient 4,43 g de diphényl4,4 (méthoxy-2 phényl)-7 [(méthoxy-2 phényl)-2 propionyl-(S)]-2 hexahydro-2,3,3a,4,5,7a-(1H)-isoindole-(3aS,7aR) sous la forme de cristaux blancs fondant à 104°C avec décomposition.

### Exemple d'utilisation 28

A une solution de 1,1 g de diphényl-7,7 (méthoxy-2 phényl)-4 perhydroisoindolediol-4,5-(3aS,4S,5S,7aS) et de 0,58 g d'acide (méthoxy-2 phényl)-2 propanoique-(S) dans 30 cm³ de dichlorométhane, refroidie à 0°C, on ajoute 30 mg d'hydrate d'hydroxy-1-benzotriazole, 0,66 g de (diméthylamino-3 propyl)-1 éthyl-3 carbodiimide et 0,6 cm³ de diisopropyléthylamine. On agite 3 heures et 30 minutes à température ambiante, ajoute 100 cm³ d'eau et 50 cm³ d'une solution aqueuse saturée de chlorure de sodium. La phase organique est décantée, séchée sur sulfate de magnésium et concentrée à sec sous pression réduite (2,7 kPa). Le résidu jaune obtenu est chromatographié sur colonne de gel de silice Merck (granulométrie 0,04-0,06 mm, diamètre 2 cm, hauteur 30 cm), en éluant sous une pression de 0,5 bar d'azote par un mélange de cyclohexane et d'acétate d'éthyle (60/40 en volumes) et en recueillant des fractions de 25 cm³. Les fractions 6 à 14 sont réunies puis concentrées à sec sous pression réduite (2,7 kPa) . On obtient 0,9 g de diphényl-7,7 (méthoxy-2 phényl)-4 [(méthoxy-2 phényl)-2 propionyl-S)]-2 perhydroisoindolediol-4,5 (3aS,4S,5S,7aS) fondant à 256°C.

### Exemple d'utilisation 29

On prépare le diphényl-7,7 (méthoxy-2 phényl)-4 [(benzyloxy-2 phényl) acétyl]-2 perhydroisoindolediol-4,5-(3aRS,4RS,5RS,7aRS) selon le mode opératoire de l'exemple d'utilisation 28, à partir de 1,24 g de diphényl-7,7 (méthoxy-2 phényl)-4 perhydroisoindolediol-4,5-(3aRS,4RS,5RS,7aRS) et de 0,8 g d'acide (benzyloxy-2) phényl acétique. On obtient 1,7 g de diphényl-7,7 (méthoxy-2 phényl)-4 [(benzyloxy-2 phényl) acétyl]-2 perhydroisoindolediol-4,5-(3aRS,4RS,5RS,7aRS) fondant à 158°C.

Un mélange de 1,5 g de diphényl-7,7 (méthoxy-2 phényl)-4 [(benzyloxy-2 phényl) acétyl]-2 perhydroisoindolediol-4,5 (3aRS,4RS,5RS,7aRs) et de 50 cm³ d'éthanol est chauffé à 60°C sous agitation; on ajoute 0,5 g d'hydroxyde de palladium sur charbon à 20% puis le mélange réactionnel est hydrogéné, sous agitation, à une température de 60°C et sous pression atmosphérique. Aprés 45 minutes de réaction, le volume théorique d'hydrogène a été absorbé; le mélange réactionnel est filtré, puis concentré à sec sous pression réduite (2,7 kPa). Le résidu est cristallisé dans 20 cm³ d'oxyde d'isopropyle. On obtient 0,8 g de diphényl-7,7 (méthoxy-2 phényl)-4 [(hydroxy-2 phényl) acétyl]-2 perhydroisoindolediol-4,5-(3aRS,4RS,5RS,7aRS) fondant à 188-190°C.

### Exemple d'utilisation 30

En opérant selon le mode opératoire de l'exemple d'utilisation 21, à partir de 0,62 g de diphényl-7,7 (méthoxy-2 phényl)-4 perhydroisoindolediol-4,5-(3aRS,4RS,5RS,7aRS) et de 0,30 g d'acide (diméhylamino-2) phényl acétique, on obtient 0,47 g de diphényl-7,7 (méthoxy-2 phényl)-4 [(diméthylamino-2 phényl) acétyl]-2 perhydroisoindolediol-4,5-(3aRS,4RS,5RS,7aRS) fondant à 250°C.

### Exemple d'utilisation 31

En opérant selon le mode opératoire de l'exemple d'utilisation 21 à partir de 1,04 g de diphényl-7,7 (méthoxy-2 phényl)-4 perhydroisoindolediol-4,5-(3aS,4S,5S,7aS) et de 0,49 g d'acide indolyl-3 acétique, on obtient 1,25 g de diphényl-7,7 (méthoxy-2 phényl)-4 (indolyl-3 acétyl)-2 perhydroisoindolediol-4,5-(3aS,4S,5S,7aS) fondant à 210°C.

### Exemple d'utilisation 32

En opérant selon le mode opératoire de l'exemple d'utilisation 21 à partir de 0,62 g de diphényl-7,7 (méthoxy-2 phényl)-4 perhydroisoindolediol-4,5-(3aRS,4RS,5RS,7aRS) et de 0,31 g d'acide (N-méthyl indolyl)-3 acétique, on obtient 0,55 g de diphényl-7,7 (méthoxy-2 phényl)-4 [(N-méthyl indolyl-3) acétyl]-2 perhydroisoindolediol-4,5-(3aRS,4RS,5RS,7aRS) fondant à 240°C.

## Revendications

1. Un dérivé de perhydroisoindole caractérisé en ce qu'il répond à la formule générale : 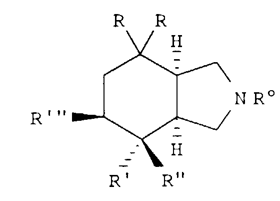 dans laquelle :
- les symboles R sont identiques et représentent des radicaux phényle éventuellement substitués par un atome d'halogène ou par un radical méthyle en position 2 ou 3,
- le symbole R' représente un radical phényle éventuellement substitué en position -2 par un radical alcoyle ou alcoyloxy contenant 1 ou 2 atomes de carbone,
- le symbole R" représente un atome de fluor ou un radical hydroxy, et le symbole R"' représente un atome d'hydrogène ou bien
- les symboles R" et R"' représentent des radicaux hydroxy, ou bien
- le symbole R" forme avec R"' une liaison, et
- le symbole R° représente un atome d'hydrogène ou représente un radical protecteur choisi parmi alcoyloxycarbonyle, benzyloxycarbonyle, benzyle éventuellement substitué, formyle, chloracétyle, trichloracétyle, trifluoroacétyle, vinyloxycarbonyle, phénoxycarbonyle, chloro-1 éthoxycarbonyle ou chlorocarbonyle,
ainsi que sa forme racémique et les mélanges avec sa forme racémique,
ainsi que ses sels lorsqu'ils existent.

2. Un dérivé de perhydroisoindole selon la revendication 1 pour lequel :
les symboles R sont identiques et représentent des radicaux phényle,
le symbole R' représente un radical phényle éventuellement substitué en position -2 par un radical alcoyle ou alcoyloxy contenant 1 ou 2 atomes de carbone,
le symbole R" représente un atome de fluor ou un radical hydroxy, et le symbole R"' représente un atome d'hydrogène ou bien les symboles R" et R"' représentent des radicaux hydroxy, ou bien le symbole R" forme avec R"' une liaison, et
le symbole R° représente un atome d'hydrogène ou représente un radical benzyle ou t.butoxycarbonyle.

3. Un dérivé de perhydroisoindole selon la revendication 1 caractérisé en ce qu'il s'agit du : diphényl-7,7 (méthoxy-2 phényl)-4 tert-butoxycarbonyl-2 perhydroisoindolol-4; diphényl-7,7 (méthoxy-2 phényl)-4 perhydroisoindolol-4; diphényl-7,7 (méthoxy-2 phényl)-4 tert-butoxycarbonyl-2 perhydroisoindolediol-4,5; diphényl-7,7 (méthoxy-2 phényl)-4 perhydroisoindolediol-4,5; benzyl-2 diphényl-7,7 (méthoxy-2 phényl)-4 perhydroisoindolediol-4,5.

4. Procédé de préparation d'un dérivé de perhydroisoindole selon la revendication 1, caractérisé en ce que l'on fait agir un composé organométallique de formule générale :
R'-M
dans laquelle R' est défini comme précédemment, et M représente le lithium, un radical MgX ou CeX₂ pour lequel X est un atome d'halogène, sur le dérivé correspondant de perhydroisoindolone de formule générale : 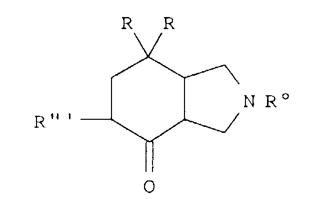 dans laquelle R est définis comme précédemment, R"' est un atome d'hydrogène ou un radical hydroxy éventuellement protégé, et R° est un radical protecteur tel que défini dans la revendication 1, puis le cas échéant libération du radical protecteur de R"', puis éventuellement transformation du produit obtenu pour lequel R" est un radical hydroxy et R"' est un atome d'hydrogène en un produit pour lequel R" est un atome de fluor et R"' est un atome d'hydrogène ou en un produit pour lequel R" et R"' forment ensemble une liaison et éventuellement élimine le radical protecteur R°, et/ou sépare les formes isomères du produit obtenu, et transforme le produit obtenu en un sel.

5. Procédé de préparation d'un dérivé de perhydroisoindole selon la revendication 1, pour lequel R" est un atome de fluor et R"' est un atome d'hydrogène, caractérisé en ce que l'on effectue la fluoration d'un dérivé de l'isoindole selon la revendication 1 pour lequel R et R' sont définis comme dans la revendication 1, R° est un radical protecteur, R" est un radical hydroxy et R"' est un atome d'hydrogène, puis éventuellement élimine le radical protecteur R° et transforme le produit obtenu en un sel.

6. Procédé de préparation d'un dérivé de perhydroisoindole selon la revendication 1, pour lequel R" et R"' forment ensemble une liaison, caractérisé en ce que l'on deshydrate le dérivé correspondant de perhydroisoindole selon la revendication 1 pour lequel R" est un radical hydroxy et R"' est un atome d'hydrogène et R et R° sont définis comme dans la revendication 1, puis éventuellement élimine le radical protecteur R° et transforme le produit obtenu en un sel.

7. Utilisation d'un produit selon la revendication 1, pour la préparation d'un dérivé de perhydroisoindole de formule générale : 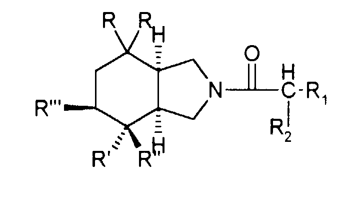 dans laquelle R, R', R" et R"' sont définis comme dans la revendication 1, le symbole R₁ représente un radical phényle éventuellement substitué par un ou plusieurs atomes d'halogène ou radicaux hydroxy, alcoyle pouvant être éventuellement substitués (par des atomes d'halogène ou des radicaux amino, alcoylamino ou dialcoylamino) alcoyloxy ou alcoylthio pouvant être éventuellement substitués [par des radicaux hydroxy, amino, alcoylamino ou dialcoylamino éventuellement substitués (par des radicaux phényle, hydroxy ou amino), ou dialcoylamino dont les parties alcoyle forment avec l'atome d'azote auquel elles sont rattachées, un hétérocycle à 5 à 6 chaînons pouvant contenir un autre hétéroatome choisi parmi l'oxygène, le soufre ou l'azote, éventuellement substitué par un radical alcoyle, hydroxy, hydroxyalcoyle)], ou substitué par des radicaux amino, alcoylamino, dialcoylamino dont les parties alcoyle peuvent former avec l'atome d'azote auquel elles sont rattachées, un hétérocycle tel que défini ci-dessus, ou représente un radical cyclohexadiènyle, naphtyle ou hétérocyclyle mono ou polycyclique, saturé ou insaturé contenant 5 à 9 atomes de carbone et un ou plusieurs hétéroatomes choisis parmi l'oxygène, l'azote ou le soufre, et éventuellement substitué par un atome d'halogène ou par un radical alcoyle ou alcoyloxy, et le symbole R₂ représente un atome d'hydrogène ou d'halogène ou un radical hydroxy, alcoyle, aminoalcoyle, alcoylaminoalcoyle, dialcoylaminoalcoyle, alcoyloxy, alcoylthio, acyloxy, carboxy, alcoyloxycarbonyle, dialcoylaminoalcoyloxycarbonyle, benzyloxycarbonyle, amino ou acylamino, sous forme racémique,
sous ses formes (R) ou (S) sur la chaîne -CHR₁R₂, ou sous forme du mélange de plusieurs de ces formes, ainsi que ses sels, étant entendu que les radicaux alcoyle ou acyle cités contiennent (sauf mention spéciale) 1 à 4 atomes de carbone en chaîne droite ou ramifiée.

## Patentansprüche

1. Ein Perhydroisoindol-Derivat, dadurch gekennzeichnet, daß es der allgemeinen Formel: 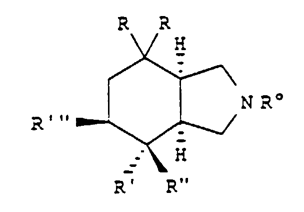 entspricht, in der
- die Symbole R gleich sind und Phenylreste darstellen, die gegebenenfalls mit einem Halogenatom oder mit einem Methylrest in 2- oder 3-Position substituiert sind,
- das Symbol R' einen Phenylrest darstellt, der gegebenenfalls in 2-Position mit einem Alkyl- oder Alkoxyrest mit 1 oder 2 Kohlenstoffatomen substituiert ist,
- das Symbol R" ein Fluoratom oder einen Hydroxyrest darstellt und das Symbol R"' ein Wasserstoffatom darstellt oder aber
- die Symbole R" und R"' Hydroxyreste darstellen oder aber
- das Symbol R" mit R"' eine Bindung bildet und
- das Symbol R° ein Wasserstoffatom darstellt oder eine Schutzgruppe darstellt, die unter einem Alkoxycarbonyl-, Benzyloxycarbonyl-, gegebenenfalls substituierten Benzyl-, Formyl-, Chloracetyl-, Trichloracetyl-, Trifluoracetyl-, Vinyloxycarbonyl-, Phenoxycarbonyl-, 1-Chlorethoxycarbonyl- oder Chlorcarbonylrest ausgewählt ist,
sowie seine Racematform und die Gemische mit seiner Racematform,
sowie seine Salze, wenn sie existieren.

2. Ein Perhydroisoindol-Derivat gemäß Anspruch 1, für das:
die Symbole R gleich sind und Phenylreste darstellen,
das Symbol R' einen Phenylrest darstellt, der gegebenenfalls in 2-Position mit einem Alkyl- oder Alkoxyrest mit 1 oder 2 Kohlenstoffatomen substituiert ist,
das Symbol R" ein Fluoratom oder einen Hydroxyrest darstellt und das Symbol R"' ein Wasserstoffatom darstellt oder aber
die Symbole R" und R"' Hydroxyreste darstellen oder aber
das Symbol R" mit R"' eine Bindung bildet und
das Symbol R° ein Wasserstoffatom darstellt oder einen Benzyl- oder tert.-Butoxycarbonylrest darstellt.

3. Ein Perhydroisoindol-Derivat gemäß Anspruch 1, dadurch gekennzeichnet, daß es sich um: 7,7-Diphenyl-4-(2-methoxyphenyl)-2-tert.-butoxycarbonylperhydroisoindol-4-ol; 7,7-Diphenyl-4-(2-methoxyphenyl)perhydroisoindol-4-ol; 7,7-Diphenyl-4-(2-methoxyphenyl)-2-tert.-butoxycarbonylperhydroisoindol-4,5-diol; 7,7-Diphenyi-4-(2-methoxyphenyl)perhydroisoindol-4,5-diol; 2-Benzyl-7,7-diphenyl-4-(2-methoxyphenyl)perhydroisoindol-4,5-diol handelt.

4. Verfahren zur Herstellung eines Perhydroisoindol-Derivats gemäß Anspruch 1, dadurch gekennzeichnet, daß man eine metallorganische Verbindung der allgemeinen Formel:
R'-M
in der R' wie vorhergehend definiert ist und M Lithium, einen MgX- oder CeX₂-Rest, für den X ein Halogenatom ist, darstellt, auf das entsprechende Perhydroisoindolon-Derivat der allgemeinen Formel: 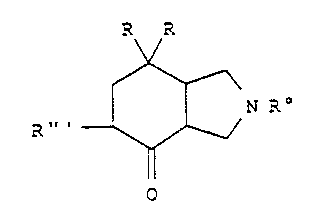 in der R wie vorhergehend definiert ist, R"' ein Wasserstoffatom oder eine gegebenenfalls geschützte Hydroxygruppe ist und R° eine wie in Anspruch 1 definierte Schutzgruppe ist, einwirken läßt, dann gegebenenfalls die Schutzgruppe von R"' freisetzt, dann gegebenenfalls das erhaltene Produkt, für das R" ein Hydroxyrest ist und R"' ein Wasserstoffatom ist, in ein Produkt, für das R" ein Fluoratom ist und R"' ein Wasserstoffatom ist, oder in ein Produkt, für das R" und R"' zusammen ein Bindung bilden, überführt und gegebenenfalls die Schutzgruppe R° entfernt und/oder die isomeren Formen des erhaltenen Produkts trennt und das erhaltene Produkt in ein Salz überführt.

5. Verfahren zur Herstellung eines Perhydroisoindol-Derivats gemäß Anspruch 1, für das R" ein Fluoratom ist und R"' ein Wasserstoffatom ist, dadurch gekennzeichnet, daß man die Fluorierung eines Isoindol-Derivats gemäß Anspruch 1, für das R und R' wie in Anspruch 1 definiert sind, R° eine Schutzgruppe ist, R" ein Hydroxyrest ist und R"' ein Wasserstoffatom ist, ausführt, dann gegebenenfalls die Schutzgruppe R° entfernt und das erhaltene Produkt in ein Salz überführt.

6. Verfahren zur Herstellung eines Perhydroisoindol-Derivats gemäß Anspruch 1, für das R" und R"' zusammen eine Bindung bilden, dadurch gekennzeichnet, daß man das entsprechende Perhydroisoindol-Derivat gemäß Anspruch 1, für das R" ein Hydroxyrest ist und R"' ein Wasserstoffatom ist und R und R° wie in Anspruch 1 definiert sind, dehydratisiert, dann gegebenenfalls die Schutzgruppe R° entfernt und das erhaltene Produkt in ein Salz überführt.

7. Verwendung eines Produkts gemäß Anspruch 1 für die Herstellung eines Perhydroisoindol-Derivats der allgemeinen Formel: 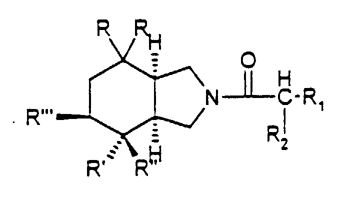 in der R, R', R" und R‴ wie in Anspruch 1 definiert sind, das Symbol R₁ einen Phenylrest darstellt, der gegebenenfalls mit einem oder mehreren Halogenatomen oder Hydroxyresten, Alkylresten, die gegebenenfalls substituiert sein können (mit Halogenatomen oder Amino-, Alkylamino- oder Dialkylaminoresten), Alkoxy- oder Alkylthioresten, die gegebenenfalls substituiert sein können [mit Hydroxy-, Amino-, Alkylamino- oder Dialkylaminoresten, die gegebenenfalls substituiert sind (mit Phenyl-, Hydroxy- oder Aminoresten), oder Dialkylaminoresten, deren Alkylteile mit dem Stickstoffatom, mit dem sie verknüpft sind, einen Heterocyclus mit 5 bis 6 Kettengliedern bilden, der ein anderes unter Sauerstoff, Schwefel oder Stickstoff ausgewähltes Heteroatom enthalten kann, der gegebenenfalls substituiert ist (mit einem Alkyl-, Hydroxy- oder Hydroxyalkylrest)], substituiert ist oder mit Amino-, Alkylamino- oder Dialkylaminoresten, deren Alkylteile mit dem Stickstoffatom, mit dem sie verknüpft sind, einen wie oben definierten Heterocyclus bilden können, substituiert ist, oder einen Cydohexadienyl-, Naphthyl-, oder einen mono- oder polycyclischen, gesättigten oder ungesättigten heterocyclischen Rest darstellt, der 5 bis 9 Kohlenstoffatome und ein oder mehrere unter Sauerstoff, Stickstoff oder Schwefel ausgewählte Heteroatome enthält und der gegebenenfalls mit einem Halogenatom oder mit einem Alkyl- oder Alkoxyrest substituiert ist, und das Symbol R₂ ein Wasserstoff- oder Halogenatom oder einen Hydroxy-, Alkyl-, Aminoalkyl-, Alkylaminoalkyl-, Dialkylaminoalkyl-, Alkoxy-, Alkylthio-, Acyloxy-, Carboxy-, Alkoxycarbonyl-, Dialkylaminoalkoxycarbonyl-, Benzyloxycarbonyl-, Amino- oder Acylaminorest darstellt, in Racematform,
in seinen (R)- oder (S)-Formen bezüglich der Kette -CHR₁R₂, oder in Form des Gemischs von mehreren dieser Formen, sowie seiner Salze, wobei es sich versteht, daß die zitierten Alkyl- oder Acylreste (außer bei spezieller Erwähnung) 1 bis 4 Kohlenstoffatome in gerader oder verzweigter Kette enthalten.

## Claims

1. A perhydroisoindole derivative, characterised in that it corresponds to the general formula: 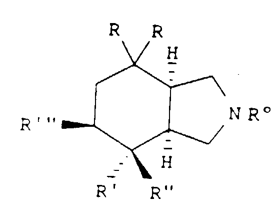 in which:
- the symbols R are identical and represent phenyl radicals which are optionally substituted by a halogen atom or by a methyl radical in position 2 or 3,
- the symbol R' represents a phenyl radical which is optionally substituted in position 2 by an alkyl or alkyloxy radical containing 1 or 2 carbon atoms,
- the symbol R" represents a fluorine atom or a hydroxyl radical and the symbol R"' represents a hydrogen atom, or
- the symbols R" and R"' represent hydroxyl radicals, or
- the symbol R" forms a bond with R"', and
- the symbol R° represents a hydrogen atom or represents a protective radical, chosen from alkyloxycarbonyl, benzyloxycarbonyl, optionally substituted benzyl or formyl, chloroacetyl, trichloroacetyl, trifluoroacetyl, vinyloxycarbonyl, phenoxycarbonyl, 1-chloroethoxycarbonyl or chlorocarbonyl,
and its racemic form and the mixtures with its racemic form, as well as its salts, where they exist.

2. A perhydroisoindole derivative according to claim 1, in which:
the symbols R are identical and represent phenyl radicals,
the symbol R' represents a phenyl radical which is optionally substituted in position 2 by an alkyl or alkyloxy radical containing 1 or 2 carbon atoms, the symbol R" represents a fluorine atom or a hydroxyl radical and the symbol R"' represents a hydrogen atom, or
the symbols R" and R"' represent hydroxyl radicals, or the symbol R" forms a bond with R"', and
the symbol R° represents a hydrogen atom or represents a benzyl or t-butoxycarbonyl radical.

3. A perhydroisoindole derivative according to claim 1, characterised in that it is: 7,7-diphenyl-4-(2-methoxyphenyl)-2-tert-butoxycarbonyl-perhydroisoindol-4-ol; 7,7-diphenyl-4-(2-methoxyphenyl)-perhydroisoindol-4-ol; 7,7-diphenyl-4-(2-methoxyphenyl-2-tert-butoxycarbonyl-perhydroisoindole-4,5-diol; 7,7-diphenyl-4-(2-methoxyphenyl)-perhydroisoindole-4,5-diol; and 2-benzyl-7,7-diphenyl-4-(2-methoxyphenyl)-perhydroisoindole-4,5-diol.

4. Process for the preparation of a perhydroisoindole derivative according to claim 1, characterised in that an organometallic compound of the general formula:
R'-M
in which R' is as defined above and M represents lithium, an MgX or CeX₂ radical, in which X is a halogen atom, is allowed to act on the corresponding perhydroisoindolone derivative of the general formula: 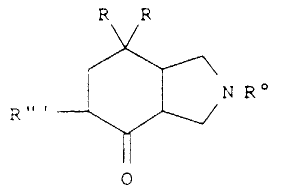 in which R is as defined above, R"' is a hydrogen atom or an optionally protected hydroxyl radical and R° is a protective radical as defined in claim 1, and, if appropriate, liberation of the protective radical of R"' and, where appropriate, conversion of the product obtained in which R" is a hydroxyl radical and R"' is a hydrogen atom into a product in which R" is a fluorine atom and R"' is a hydrogen atom, or into a product in which R" and R"' together form a bond, and/or the isomers of the product obtained are separated, and, where appropriate, the protective radical R° is removed, and the product obtained is converted into a salt.

5. Process for the preparation of a perhydroisoindole derivative according to claim 1, in which R" is a fluorine atom and R"' is a hydrogen atom, characterised in that fluorination of an isoindole derivative according to claim 1 in which R and R' are as defined in claim 1, R° is a protective radical, R" is a hydroxyl radical and R"' is a hydrogen atom is carried out and, if appropriate, the protective radical R° is then removed and the product obtained is converted into a salt.

6. Process for the preparation of a perhydroisoindole derivative according to claim 1, in which R" and R"' together form a bond, characterised in that the corresponding perhydroisoindole derivative according to claim 1 in which R" is a hydroxyl radical and R"' is a hydrogen atom and R and R° are as defined in claim 1 is dehydrated, and, if appropriate, the protective radical R° is then removed and the product obtained is converted into a salt.

7. Use of a product according to claim 1 for the preparation of a perhydroisoindole derivative of the general formula 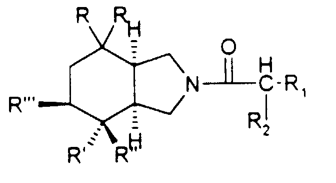 in which R, R', R" and R"' are as defined in claim 1 and the symbol R₁ represents a phenyl radical which is optionally substituted by one or more halogen atoms, hydroxyl radicals, alkyl radicals, which may be optionally substituted (by halogen atoms or amino, alkylamino or dialkylamino radicals), alkyloxy radicals or alkylthio radicals, which may be optionally substituted [by hydroxyl radicals, amino radicals, alkylamino radicals or dialkylamino radicals which are optionally substituted (by phenyl radicals, hydroxyl radicals or amino radicals), or dialkylamino radicals, the alkyl parts of which, together with the nitrogen atom to which they are attached, form a heterocyclic ring which has 5 to 6 chain members and can contain another heteroatom chosen from oxygen, sulphur or nitrogen, optionally substituted by an alkyl radical, hydroxyl radical or hydroxyalkyl radical)], or substituted by amino radicals, alkylamino radicals, dialkylamino radicals, the alkyl parts of which can form, together with the nitrogen atom to which they are attached, a heterocyclic ring as defined above, or represents a cyclohexadienyl radical, naphthyl radical or mono- or polycyclic, saturated or unsaturated heterocyclic radical containing 5 to 9 carbon atoms and one or more heteroatoms chosen from oxygen, nitrogen or sulphur, and optionally substituted by a halogen atom or by an alkyl or alkyloxy radical, and the symbol R₂ represents a hydrogen or halogen atom or a hydroxyl, alkyl, aminoalkyl, alkylaminoalkyl, dialkylaminoalkyl, alkyloxy, alkylthio, acyloxy, carboxyl, alkyloxycarbonyl, dialkylaminoalkyloxycarbonyl, benzyloxycarbonyl, amino or acylamino radical, in the racemic form,
in its (R) or (S) forms on the chain -CHR₁R₂, or in the form of a mixture of several of these forms, and of its salts, it being understood that the alkyl or acyl radicals mentioned above contain (unless specifically stated otherwise) 1 to 4 carbon atoms in a straight or branched chain.
